# EUROPEAN PATENT SPECIFICATION

(11) **EP 1 368 075 B1**
(45) Date of publication and mention of the grant of the patent: **12.04.2006**
(21) Application number: 02703423.0
(22) Date of filing: 28.02.2002
(51) Int. Cl.: A61L 33/00, A61F 2/06, B29C 59/14

(54) **PLASMA SURFACE GRAFT PROCESS FOR REDUCING THROMBOGENICITY**
PLASMA OBERFLÄCHEN-PFROPFPROZESS ZUR THROMBOGENITÄTS-REDUZIERUNG
PROCEDE DE GREFFE SUPERFICIELLE DE PLASMA PERMETTANT DE REDUIRE LA THROMBOGENICITE

(30) Priority: 02.03.2001 US 272477 P
(43) Date of publication of application: 10.12.2003
(73) Proprietor: Université Laval, Québec, Québec G1K 7P4 (CA)
(72) Inventor: LAROCHE, Gaétan, Saint-Augustin-de Desmaures, Quebec G3A (CA); MANTOVANI, Diego, Quebec, Quebec G1K 2P2 (CA); CHEVALLIER, Pascale, Quebec, Quebec G1R 1X9 (CA); CASTONGUAY, Martin, Cap-Rouge, Quebec G1Y 2B8 (CA); PAGEAU, Jean-François, Ste-Julie, Quebec J3E 2K2 (CA)
(74) Representative: Guerre, Dominique
(86) International application number: PCT/CA2002/000261
(87) International publication number: WO 2002/070032

(56) References cited:
- EP-A- 0 519 087
- US-A- 4 720 512
- US-A- 5 914 115
- SIPEHIA R: "X-ray photoelectron spectroscopy studies, surface tension measurements, immobilization of human serum albumin, human fibrinogen and human fibronectin onto ammonia plasma treated surfaces of biomaterials useful for cardiovascular implants and artificial cornea implants." BIOMATERIALS, ARTIFICIAL CELLS, AND IMMOBILIZATION BIOTECHNOLOGY: OFFICIAL JOURNAL OF THE INTERNATIONAL SOCIETY FOR ARTIFICIAL CELLS AND IMMOBILIZATION BIOTECHNOLOGY. UNITED STATES 1993, vol. 21, no. 5, 1993, pages 647-658, XP001084777 ISSN: 1055-7172 cited in the application
- SIPEHIA R: "The enhanced attachment and growth of endothelial cells on anhydrous ammonia gaseous plasma modified surfaces of polystyrene and poly(tetrafluoroethylene)." BIOMATERIALS, ARTIFICIAL CELLS, AND ARTIFICIAL ORGANS. UNITED STATES 1990, vol. 18, no. 3, 1990, pages 437-446, XP001095207 ISSN: 0890-5533

## Description

### FIELD OF THE INVENTION

The present invention relates to a novel process for modifying the surface properties of a material that is suitable for contact with living tissue comprising: Exposing the surface of the material to plasma treatment conditions in order to create reactive groups on said surface; activating a molecule to produce a reactive molecular species capable of forming strong bonds with the reactive groups created on the surface of the material; and contacting the reactive molecular species with the reactive groups created on the surface of the material to form strong bonds. The invention further encompasses the materials produced by this process as well as devices that are comprised of these process-modified materials, such as vascular prostheses.

### BACKGROUND OF THE INVENTION

Approximately 350 000 synthetic vascular prostheses are implanted each year as arterial bypasses in Western countries and Japan. Made of Dacron™ (polyethyleneterephthalate) or microporous Teflon™ (expanded polytetrafluoroethylene, or ePTFE), they perform quite well in large-diameter vessels under high flow conditions but have a low patency rate when used as small-diameter bypasses (i.e., coronaries) or as medium-diameter bypasses in low-flow conditions and high-resistance locations (i.e., leg arteries). (The patency rate relates to a biomaterial's ability to remain pervious to blood flow when replacing an artery). A study on 398 ePTFE vascular prostheses implanted as medium-diameter bypasses and retrieved following complications revealed that 65 % of them had to be explanted due to thrombosis.⁽¹⁾

Endothelial cell lining is the natural blood-compatible surface that covers the inside surface of blood vessels and heart chambers, and it is recognized to be the best possible hemocompatible surface. The principal techniques that have been proposed in the past to promote endothelial cell adhesion and spreading onto the luminal surfaces of vascular prostheses are: (1) endothelial cell seeding; (2) graft pre-treatment with endothelial cell mitogens; (3) increasing structural porosity; and (4) coating the surface with a protein matrix. While several attempts have been made, the complete endothelial cell coverage of the blood-exposed inside prosthetic surfaces has never been observed in humans. It is therefore evident that increasing the patency rate of arterial prostheses is a high priority in current cardiovascular research.

It has been shown in many instances that surface modification through plasma treatment is one of the most promising techniques that may be used to improve specific hemocompatibility and general biocompatibility of polymeric materials.^{(2,3)} One advantage of the plasma treatment is that it allows formation of a covalent bond between the modifier and the surface to be treated, unlike other approaches where a coating is deposited inside the prosthesis without formation of a covalent bond ^{(4,5)} , where the bond is ionic ⁽⁶⁾ or where the bioactive substance is incorporated in the biomaterial. ⁽⁷⁾ Following to the covalent bonding of the modifier to the surface of the material, made possible by plasma treatment, a molecule chosen for its biocompatibility properties can be covalently attached to the modifier either directly or through a spacer molecule. Clinically, the increased stability of a surface treatment involving plasma treatment could result in improved limb salvage rates and a possible future use in small-diameter coronary bypass operations. This, in turn, could minimize the incidence of re-operation for patients and lower social healthcare costs related to surgical interventions of this type.

Plasma treatment of biomaterials is not the only process that can yield covalent bonding of molecules possessing desirable hemocompatibility and/or biocompatibility. However, the processes that are described in the relevant prior art frequently involve several steps.⁽⁸⁻¹⁴⁾ To date, most of the experiments performed to improve the performance of biomaterial surfaces have been based on flat specimens of material, particularly polymeric sheets. Unfortunately, few experiments involving the use of plasma to activate the surface have been carried out directly on commercial prostheses with a non-planar geometry. Experiments done at the University of Washington,^{(2,15-20)} which consisted mainly in treating commercial Dacron™ arterial prostheses with a plasma treatment in a tetrafluoroethylene (TFE) gas environment so as to coat the internal surfaces of the prostheses with potentially more biocompatible CF₃ groups, were a precursor to the development of the new commercial Radio Frequency Glow Discharge (RFGD)-treated vascular grafts.⁽¹⁷⁾ Yet, despite the existence of many surface treatment systems for biomaterial surfaces, few have resulted in the *treatment,* as opposed to the *coating,* of the internal surface of tubular devices.⁽²¹⁻²³⁾

Over the last 30 years, a number of studies have been designed to test the ability of plasma to either treat or coat the surfaces of biomedical devices with a view to enhancing their biocompatibility or to modulate the interactions of polymers and tissue when prostheses are implanted *in situ*.^{(16,24-30)} Plasma polymerizable gas has been used to coat various substrates with a thin polymeric layer,^{(15,17-19,24,31,32)} while non-polymerizable gas has been applied *to treat* substrate surfaces. ⁽³³⁻³⁸⁾

Despite the fact that many approaches have been developed to coat or treat the surface of biomaterials, the introduction of amino groups through RFGD treatment is particularly interesting for two reasons. First, amino groups are known to facilitate cellular spreading on a biomaterial surface.⁽³⁹⁻⁴¹⁾ Second, amino groups react readily with other chemical functional groups, allowing for the attachment of specific molecules through interactions sufficiently strong to prevent the leaching of these molecules by the blood stream.⁽⁴²⁾ For example, amino groups can be used to ionically immobilize heparin, a well-known polysaccharide anticoagulant.⁽²²⁾

Several patents describe process in which amino groups are involved.

For example, it is known from US 4 720 512, a process for reducing thrombogcnecity of polymeric articles in contact with blood by exposing the pretreated surface of the polymer with an amine-rich material to plasma treatment, then grafting aldehyde activated heparin covalcntly to the pretreated surface. This attachment of anti-thrombogenic agents to the surface of a vascular graft is achieved, but the process implies a step of using both a polymer and a prepolyner in order to obtain amine groups.

It is known from US 5 914 115 a radio frequency plasma coating no less of medical devices with covalently bond heparin in which, amine groups are introduced into the surface by RF plasma and then heparin is covalently bound.

It is also known from EP 0 519 087, another method to bound a biological antithroinbotic agent onto the surface of a medical device. This method comprises a plasma polymerisation of a monomer with amine groups onto the medical device followed by the grafting of the biological agent.

Despite the above described methods and processes, there is still a need of alternative method to covalently attach anti-thrombogenic agent onto the surface of medical devices.

While many efforts have been made in the last 20 years in the field of cardiovascular prosthetic devices to develop surfaces with improved human blood compatibility, an acceptable long-term blood-compatible synthetic material has yet to be achieved. The complexity of the interactions between blood and synthetic materials constitutes a major interfacial problem in this respect.

### SUMMARY OF THE INVENTION

Surface treatments allow a modulation of the surface properties of a biomaterial to enhance its interfacial reaction with a biological environment. Low pressure plasma surface treatments are particularly advantageous in the design and development of new biocompatible materials, since they permit surface modifications without altering the hulk of the materials' properties.⁽⁴³⁾ In the context of the present invention, they arc particularly useful for modulating different tissue/biomaterial interface properties, but their ultimate utility may reside in their significant improvement of the hemocompatibility of vascular prostheses.

Thus, in accordance with the present invention, there is provided a novel process for modifying the surface properties of a material that is to be placed in contact with living tissues. This process comprises:
-- Exposing the surface of the material to plasma treatment conditions in order to create reactive groups on the surface of the material;
-- Activating a molecule with an activator to produce a reactive molecular species capable of forming strong bonds with the reactive groups created on the surface of the material; and
-- Contacting the reactive molecular species with the reactive groups created on the surface of the material to form strong bonds.

Preferably, the strong bonds formed are covalent bonds. In a preferred embodiment, the molecule selected for activation is compatible with living tissue, including human tissue.

In a specific embodiment the novel process of the present invention further comprises the following features:
-- the plasma is ammonia Radio Frequency (RF) plasma and the reactive groups are amine groups;
-- the molecule selected for activation is chosen from the group consisting of choline, heparin, and other molecules known to those skilled in the art for their hemocompatibility, the activator is phosphoryl chloride (POCl₃) and the reactive molecular species is the oxyphosphorodichlorinated derivative of the molecule; and
-- the strong bonds are phosphoamide-type covalent bonds.

In a preferred embodiment of the specific embodiment above, the RF power is between about 5 watts to about 500 watts and is applied for a time of about 10 seconds to about 30 minutes at a pressure of about 6 661 Pa (50 mtorr) to about 666,1 Pa (5 torr). Those skilled in the art will recognize that care has to be taken in order to avoid combinations of time and power that would deliver excessive amounts of energy to the treated surface. Such conditions lead to a microscopically brittle surface. Preferably, the RF power is about 20 watts and is applied for a time of about 250 seconds at a pressure of about 39,966 Pa (300 mtorr.) In yet another preferred embodiment, the RF power is about 15 watts and is applied for a time of about 100 seconds at a pressure of about 83,305 Pa (250 mtorr.)

Ideally, the third step of the process is performed within about 2 hours of the first step.

The novel process of the present invention is particularly suitable for an implantable material selected from the following group: expanded polytetrafluoroethylene (cPTFE), polytetrafluoroethylene (PTFE), polyathylenes, polyesters, polypropylenes and polyurethanes. In a preferred embodiment, the implantable material is ePTFE, and the treated surface is the internal surface of a vascular prosthesis. In a more preferred embodiment, the vascular prosthesis has an inner diameter of about 1 to 30 mm.

The present invention further comprises the materials produced by the novel process, as well as devices comprising these materials, including any of the following: an implant, a prosthesis, an artificial organ, a stent, a cardiac valve, an apparatus contacting blood during an extra-corporal blood circulation, an apparatus contacting blood during a dialysis treatment or any other material with surfaces coming in contact with blood.

Additionally, the present invention is meant to cover the use of any such device, particularly to prevent thrombosis in an animal, including a human being.

By controlling the RF power, the ammonia pressure, and the treatment duration, the atomic substitution percentage may be modulated and up to 15% of the surface atoms may be substituted with nitrogen, as probed by X-ray photoelectron spectroscopy (XPS). On the surface, different chemical species are present after the treatment, such as amine and imine groups. Storage in air for up to 80 days shows a defluorination of the surface and a slow decrease of the surface nitrogen concentration during this storage. Ammonia RF Plasma treatment can be successfully used to uniformly treat the internal surface of an ePTFE arterial prosthesis despite the fact that care has to be taken to prevent a significant surface reorganization upon exposure to the atmosphere.^{(44,45)}

The ammonia RF plasma-treated internal surfaces of vascular prostheses may be reacted with a substance whose molecular structure can render the prostheses more hemocompatible and non-thrombogenic when in use. The molecule is preferably activated phosphorylcholine (PRC) or any other molecule with similar properties, wherein the two hydroxyl groups on the phosphate moiety have been substituted with chlorine to form the dichloro derivative of the molecule. This dichloro derivative readily reacts with the amino groups resulting from the ammonia RF plasma treatment of the inside surfaces of the vascular prostheses, allowing for the effective anchoring of the molecule.

It is an object of the present invention to provide a novel process for producing a material suitable for contact with a living tissue. This material is characterized in having a surface that is resistant to neutrophil adhesion and thrombosis but that enhances the development of fibroblasts and endothelial cells. The process may be used, for example, to treat the inside surfaces of devices, such as vascular prostheses, so as to enhance their hemocompatibility and make them less thrombogenic when in use. A further object of the invention is therefore to provide devices treated with the novel process of the present invention.

Other objects, advantages and features of the present invention will become more apparent upon reading of the following non restrictive description of preferred embodiments thereof, given by way of example only with reference to the accompanying drawings.

### BRIEF DESCRIPTION OF THE FIGURES

**Figure 1** is a schematic representation of the Radio Frequency Glow Discharge treatment system used in the process of the invention.
**Figure 2** shows the XPS spectrum of the inside surface of an untreated ePTFE vascular prosthesis and the XPS spectrum of the inside surface of an ammonia plasma treated ePTFE vascular prosthesis.
**Figure 3** shows the effect on the surface composition of treatments performed at 39,966 Pa (300 mtorr) of ammonia by varying the power (20, 40 and 50 W) and treatment times (10, 120 and 275 seconds).
**Figure 4** shows the relationship between the pressure within the chamber and the modification of the ePTFE prosthetic surface.
**Figure 5** shows the percentage of nitrogen and the N/C and F/C ratios measured on the surface as a function of ammonia plasma treatment time.
**Figure 6** shows the derivatization reaction of a plasma treated PTFE film with chlorobenzaldehyde.
**Figure 7** shows the relationship between the atomic surface concentration of phosphorous grafted onto the surface (%P) and the intensity of the N' peak at 402 eV.
**Figure 8** shows the cellular growth determined by the fluorescence of DNA strands using specific markers with untreated and treated prostheses as well as with "control" cell cultures.
**Figure 9** reproduces SEM micrographs showing the degree of accumulation of blood platelets within (a) a prosthesis treated in accordance with the process of the invention and (b) an untreated prosthesis.
**Figure 10** shows the quantification of the adherent blood platelets on untreated and treated prostheses.
**Figure 11** shows the quantification of the adherent neutrophils on untreated and treated prostheses.
**Figure 12** shows the influence of a treated and untreated prostheses on the clotting time.
**Figure 13** shows the influence of a treated and untreated prostheses on the thrombogenic index.
**Figure 14** displays microscopic slides of the growth of fibroblasts within a prosthesis treated in accordance with the process of the invention **((a)** and **(c))** and an untreated prosthesis **((b)** and **(d)).**
**Figure 15** SEM images at 20x and 2000x magnification for ANBIOPA™-treated prostheses and commercially-available untreated prostheses after a one-month implantation in a canine model: **a)** ANBIOPA™-treated, 20x; **b)** untreated, 20x; **c)** ANBIOPA™-treated, 2000x; and **d)** untreated, 2000x.

### DESCRIPTION OF A PREFERRED EMBODIMENT

For the present purposes, we give the following definitions of the terms "untreated" and "treated". "Untreated" means a commercially available vascular prosthesis, to which the inventors did not apply any surface modification treatment. "Treated" means a commercially available vascular prosthesis to which the inventors applied the surface modification procedure hereafter described.

The term "animal" as used herein is meant to signify human beings, primates, domestic animals (such as horses, cows, pigs, goats, sheep, cats, dogs, guinea pigs, mice, etc.) and other mammals. Generally, this term is used to indicate living creatures having highly-developed vascular systems.

### I. Pre-Treatment of the Inside Surface of Vascular Prostheses with Ammonia Radio Frequency (RF) Plasma

### Experimental Method

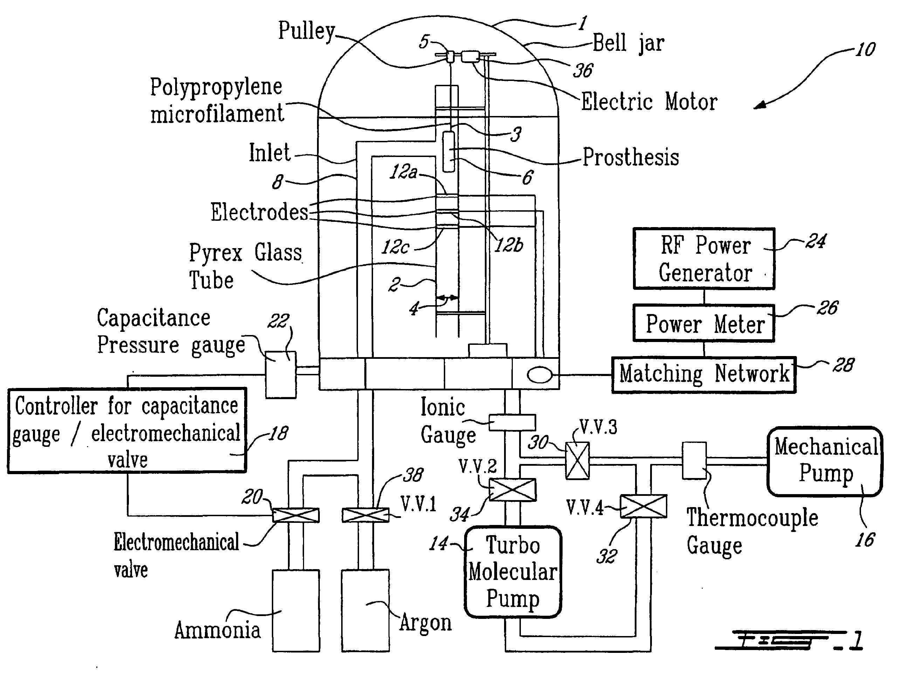

(The Experimental Method is described with reference to Figure 1.)

### Materials

Microporous ePTFE vascular prostheses **6** (6 and 10 mm ID) with a fibril average length of 25 microns were used, and the method is described in relation to a single prosthesis.

A microporous ePTFE arterial prosthesis (length 3 to 10 cm) was first washed with high purity methanol for 10 minutes and vacuum-dried. This procedure was performed to remove any contaminants which may have been present on the surface of the "as-received" prosthesis. Following this washing procedure, two small holes were punctured at one end of the prosthesis, facing each other, and a polypropylene monofilament (5-0 - Ethicon) **3** was inserted through both holes and a knot tied loosely above the prosthesis. The other end of the monofilament was rolled around a pulley **5,** itself located on the horizontal shaft of an electric motor **36** to allow a vertical displacement of the sample prosthesis inside the Pyrex™ tube **2** of the RFGD treatment system. Gravity caused the vascular prosthesis to move down inside the Pyrex™ tube. For vascular grafts longer than 3 cm, it was preferable, in addition to the aforementioned knot and its attachment via the monofilament to the pulley, to attach a polypropylene monofilament **3** at the bottom of the vascular prosthesis and to attach the monofilament to the pulley by first letting the monofilament run down the tube and then up to the pulley. With this set-up, the motor can pull on the prosthesis when it is going down the Pyrex™ tube, and therefore friction of the prosthesis on the inside wall of the tube ceases to be a problem when moving the prosthesis down the tube. Prior to closing the system, a container and its cap were placed inside a bell jar **1** to collect and store the prosthesis in an argon atmosphere following the RFGD treatment, thereby minimizing any contact of the active sites on the surface of the prosthesis with air.

### Plasma Equipment

As shown in Figure 1, the RFGD treatment system used can be generally represented by the numeral **10**. This system is basically comprised of a Pyrex™ glass tube (400 mm long, 12.5 mm i.d. for 10 mm i.d. prostheses and 8 mm i.d. for 6 mm i.d. prostheses) **2** with an internal diameter **4** slightly greater than the external diameter of the prosthesis **6**, a configuration which allows for RFGD generation on the internal side of the prosthesis only. Indeed, the path length between the external side of the prosthesis **6** and the Pyrex™ tube **4** is not long enough to allow the election-induced cascade ionization which is necessary for plasma formation.⁽¹¹⁾ Therefore, the treatment of prostheses of various diameter requires the selection of a Pyrex™ tube of the appropriate diameter.

As mentioned above, the criteria of selection is that no plasma is ignited on the outside surface of the prosthesis during its treatment. A space of about 1 mm all around the outside diameter of the prosthesis and the inside wall of the Pyrex™ glass tube **2** has proven to be appropriate. The Pyrex™ glass tube **2** has an inlet for gas injection **8** located 5 cm below its upper end. Three capacitively coupled copper electrodes **12a, 12b** and **12c,** spaced from 3 to 5 cm from each other (depending on the length of the prosthesis to treat), are attached to the middle of the tube **2.** The vacuum within the chamber was created by a turbo-molecular pump **14** with a pumping speed of 60 liters/s (Model TCP015, Balzers Pfeiffer) which, in turn, is connected to a mechanical pump **16** (Model 1402, Welch Scientific Company). This set-up allows a pressure of close to 532,38 10⁻⁵ Pa (4 x 10⁻⁵ torr) to be reached. During treatment, pressure is kept constant within the chamber by means of a capacitance pressure gauge/electromechanical valve controller **18** (type 250-1A, MKS Instruments) connected to an electromechanical valve **20** (type 248A-00200SV, MKS Instruments) and a capacitance manometer **22** (Baratron type 626A01TAE, MKS Instruments). Finally, gaseous moleculcs are excited through a radio-frequency field made by an RF generator **24** (YAESU HF transceiver FT-840, YAESU) that transmits the power, through a power meter **26,** to an automatic matching network (Smartuner SG-230, SGC) **28** which then couples the RP power with an antenna made of three copper electrodes **12a-c.** Electrodes **12a** and **12c**, located at both ends of the antenna, are connected to a ground (not shown) while the RF power is brought through the central electrode **12b**. The treatment can be operated in a RF power range from 10 to 100W at 13.56 MHz.

### Plasma Treatment

Initially, all valves on the system arc closed. After the introduction of the prosthesis into the bell jar **1,** valve **30** is opened and the mechanical pump **16** is activated to reach a vacuum of approximately 133,22 10⁻³ Pa (10⁻³ torr). As soon as this pressure is attained inside the system, valve **30** is closed, valve **32** is opened and after a few seconds the turbo-molecular pump **14** is activated. After about one minute, valve **34** is opened and pumping inside the bell jar continued until it reaches a vacuum 666,1 10⁻⁹ Pa greater than (5 x 10⁻³ torr) The chamber is then isolated from the turbo pump **14** by closing valve **34** and high purity ammonia is introduced into the bell jar **1** via electromechanical valve **20** and, when the pressure is above 0.1 torr, the bell jar **1** is pumped by the mechanical pump **16** alone, through valve **30.** The electromechanical valve controller **18** automatically regulates the amount of gas entering the chamber and the pressure, read by the capacitance gauge **22,** settles within 1 minute to the value selected on the capacitance gauge/elcctromechanical valve controller. Typically, this configuration allows plasma generation within a pressure range of 133,22 10⁻³ Pa (10⁻³) to 133,22 Pa (1) torr.

With the above parameters established, the plasma is ignited by turning the radio-frequency generator **24** on. Sufficient time is allowed for the matching network **28** to reach a standing wave ratio (SWR) signal of between 1 and 1.15, as read on the power meter **26** in order to provide optimal matching of the power input to the discharge. RFGD treatments are then performed using a static treatment: the prosthesis **6** is initially located just under the inlet **8** while the plasma parameters are adjusted, it is then moved from this position in the Pyrex™ glass tube **2** into the middle of the lighted area (between the three electrodes **12a, 12b** and **12c)** using the electric motor **36,** and is maintained within the plasma for pre-determined periods of time. Prostheses longer than 10 cm could be treated by slowly moving the prostheses across the lighted area using electric motor 36, resulting in similar grafting efficiencies. ⁽⁴⁵⁾

At the end of the treatment, the Radio-Frequency generator **24** is turned off and the gas flow stopped by closing the electromechanical valve **20** via its controller **18.** The mechanical pump **16** is kept in operation to remove residual gaseous ammonia from the bell-jar **1** until a pressure of approximately 50 mtorr is reached. It is then flushed with argon for 2 minutes by carefully opening valve **38.** This is done in order to allow the excited species resulting from the plasma treatment to reach their ground state. After isolating the mechanical pump **16** from the bell-jar by closing valve **30,** argon flow is continued inside the chamber to bring the bell jar back to atmospheric pressure. The prosthesis **6** is then lowered to the bottom of the Pyrex™ tube **2,** the bell jar **1** is quickly opened and the prosthesis **6** is disconnected from the polypropylene microfilament **3** and put inside a capped container. It is then placed in a glove box purged with nitrogen where a small sample is taken for XPS studies in order to assess the success of the ammonia treatment. The prosthesis is then prepared for the anchoring process as described below.

### II. Anchoring of Molecules

Mimicry of the non-thrombogenic surface of the erythrocyte has been advocated as the starting point for the development of non-thrombogenic biomaterials. Since phosphorylcholine (PRC) is a major component of the outer surface of the erythrocyte, materials containing it would be expected to be non-thrombogenic.⁽⁴⁶⁻⁵⁰⁾ Moreover, it is known that PRC groups attached to polymer surfaces improve hemocompatibility. ⁽⁵¹⁻⁵⁷⁾

In order to enhance the hemocompatibility and non-thrombogenicity of the interior surfaces of vascular prostheses, prostheses which had been pre-treated with plasma (as described above) were made to react with activated analogues of molecules such as phosphorylcholine (PRC). Since the ultimate goal is the implantation of such prostheses into the human body, the molecules must be solidly attached to the inside walls of the prostheses. Consequently, it is necessary to form strong bonds (preferably, covalent bonds) between the molecules and the amine groups created by the plasma treatment rather than to rely on the formation of ionic bonds or adsorption phenomenon to secure the molecules onto the inside surfaces of the prostheses. Additionally, it is desirable that any chemical treatment of this type be simple and inexpensive.

The general structure of the molecules is represented below :

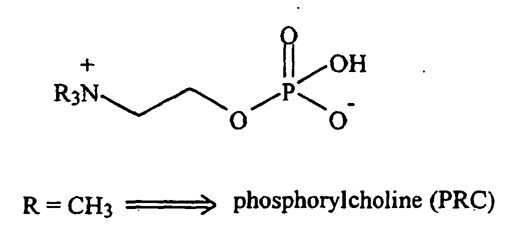

Direct reaction between PRC and the amino groups present on the surface of the plasma-treated prostheses results in ionic bonds and/or surface adsorption neither of which, as previously stated, is sufficiently strong or stable for the applications contemplated. It is therefore necessary to enhance the reactivity of these molecules by synthesizing reactive analogues so that they may partake in the creation of phosphoamide-type covalent bonds with the amino groups on the surface of the ammonia plasma pre-treated prostheses. In this way, the molecules may be anchored effectively to the interior surface of the prostheses.

In order to achieve the desired covalent reaction, the dichlorinated derivative RPO(Cl)₂ of the target molecule RPO(OH)₂ must first be synthesized. The synthesis of the dichlorinated derivatives of the molecules suitable for reaction with the amino groups on the inside surface of the pre-treated vascular prostheses may be achieved in at least two ways, as revealed diagrammatically below with PRC. In the first pathway, PRC, which is available commercially in the form of a calcium salt, is reacted with an acidic resin ⁽⁵⁸⁾ to eliminate the calcium. The product is then made to react with phosphorus trichloride or pentachloride (or any halogenation agent such as phosgene, oxalyl chloride, thionyl chloride) to obtain the dichlorinated derivative. An alternative synthesis is shown in the second pathway. Here, the dichlorinated derivative of PRC is synthesized directly from phosphoryl chloride and choline in a single step.

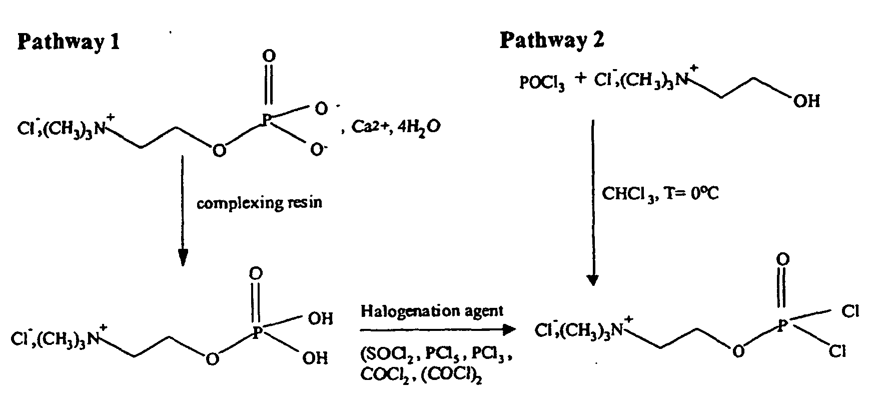

In a preferred embodiment, the preferred pathway is the second one described above which has the advantage of requiring a lesser number of steps. This experimental protocol takes into account such factors as reproducibility and toxicity, as well as feasibility on an industrial scale.

### Experimental Method

The dichloro derivative of PRC is synthesized by the addition of phosphoryl chloride (POCl₃) in chloroform solution to a chloroform solution containing choline with glass beads (3 mm diameter) at 0°C (ice bath) in a closed glass flask, under a dry nitrogen stream to exclude the presence of water and displace the HCl resulting from the reaction. The mixture is then stirred at 0°C for 1hr, and at room temperature for 12 hours to allow the reaction to proceed, as indicated by dissolution of the choline crystals and the formation of a oily phase. A slight excess of choline is used to ensure that all the phosphoryl chloride is consumed during the activation reaction. Immediately following the synthesis of the dichloro derivative of PRC, the 3 cm prostheses are immersed at room temperature in the abovementioned glass flask and agitation is continued for an additional 2 hours. In the case of longer vascular prostheses (>3 cm) a simple system was designed with a 1-liter commercial glass reactor with an outlet at the bottom. In this case, following the 12 hours of mechanical agitation, the chloroform layer at the bottom was removed via the bottom outlet of the flask and was replaced by fresh chloroform in order to remove any remaining POCl₃. A PTFE diaphragm pump is then used to circulate the activated PRC solution from the bottom of the reactor, through a section of PTFE tubing, then through a vertical glass tube in which the prosthesis is held. Finally, a section of PTFE tubing brings back the solution in the glass reactor for recirculation.

When the grafting of the activated PRC has occurred, the prostheses are washed many times in water to eliminate all traces of chlorine that may remain on the phosphoamide moiety of the anchored molecule (see Step 2 of the complete reaction, below) as well as all traces of reactants and solvent. The prostheses are then dried under vacuum overnight before being analyzed by XPS and static SIMS to determine the level of grafting.

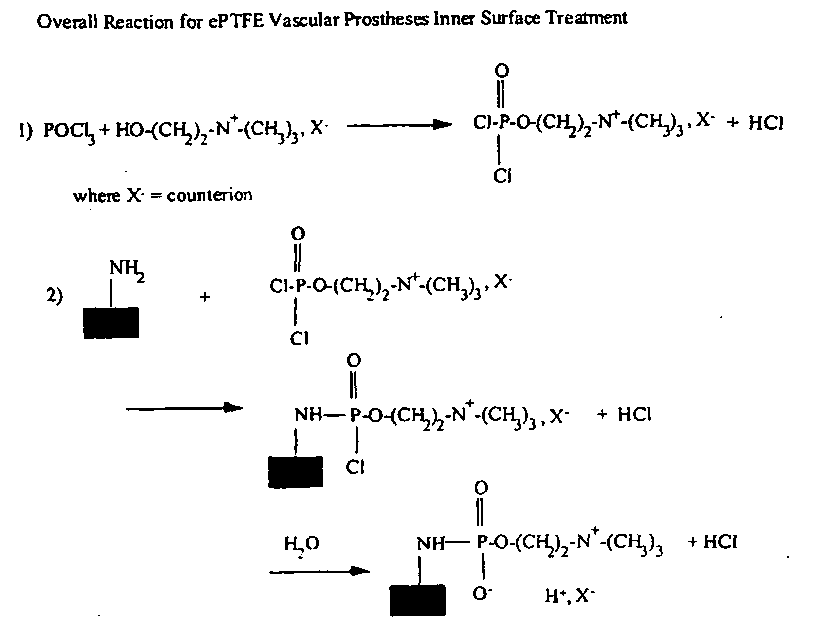

The process of the present invention may be extended to bind other molecules that are highly compatible with biological tissue, such as blood tissue. In fact, preliminary results ⁽⁶³⁾ indicate that the internal surface of commercial ePTFE arterial prostheses that were previously treated with the ammonia plasma treatment described herein can be homogeneously coated, using a grafting method different from the one presented here, with polyethylene-glycol (PEG), which is another highly biocompatible molecule.

### Characterization of ammonia RFGD plasma treated surfaces and PRC-grafted surfaces

### X-ray Photoelectron Spectroscopy (XPS) Analyses

X-ray photoelectron spectroscopy (XPS) was used to characterize the modified surfaces. XPS spectra were recorded using a PHI 5600-ci spectrometer (Physical Electronics). A monochromatic aluminum X-ray source (1486.6 eV) was used to record the survey and high resolution spectra of the prosthetic surface. For both typo of analyses, the power source was kept at 100 W to minimize X-ray induced modifications of the specimen and the detection was performed at 45° with respect to the surface. The pressure within the XPS analytical chamber was kept at about 666,1 10⁻⁵ Pa (5 x 10⁻⁹ torr)

### Ammonia RFGD plasma treated ePTFE surfaces

After RFGD ammonia plasma treatments, specimens were longitudinally opened in a chamber under nitrogen positive pressure and their internal surfaces were analyzed by XPS within 30 minutes of the RFGD treatment. Surface atomic concentration of oxygen was always lower than 5 %.

In order to collect data of the surface modifications along the longitudinally-treated length of a specimen, the XPS analysis area was moved along the length of the prosthesis. To validate that the RFGD system resulted in the treatment of the internal surface only, XPS analyses of the external surface of a prosthetic specimen treated at 20 W, for 250 seconds with an ammonia pressure of 300 mtorr were conducted. The data (not shown) confirmed that the external surface was not modified by the glow discharge when the internal diameter of the Pyrex™ tube was chosen to be only slightly larger (1 mm) than the external diameter of the prosthesis. Under the pressure and power conditions used, the Debye length did not allow the formation of a stable plasma between the external surface of the prosthesis and the internal surface of the tube, because the ionization processes were insufficient to compensate for the recombination processes.⁽⁶⁰⁾

All results are discussed by considering the percentage of atomic concentration of nitrogen on the surface, and the N/C, F/C and O/C surface concentration ratios. It was observed that because of experimental and instrumental uncertainties, atomic concentration values above 5 % are reliable to ± 1 % (absolute) or better. As gaseous ammonia was used to create the plasma, it is clear that the percentage of nitrogen would be a valid indicator of the extent of the RFGD treatment. Moreover, the N/C ratio probes the extent of nitrogen atom insertion onto the ePTFE surface, while the F/C ratio monitors the level of defluorination and is indicative of the amount of fluorine atoms substituted by other atoms and/or multiple bond formations within or between the chains.

### Relationship between the Nature of the Nitrogen-Containing Species Grafted onto the Surface of the Vascular Prostheses and Treatment Parameters

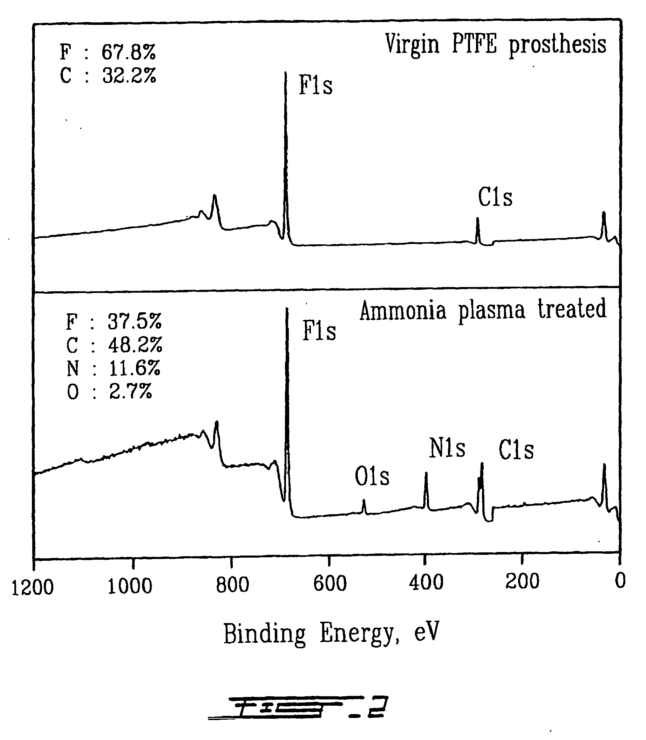

With a view to understanding the relationship between the nature of the nitrogen-containing species grafted onto the surface of the RFGD treated ePTFE samples, different tests were performed to investigate the effects of variable parameters such as gas pressure, RF power and treatment time. Prostheses measuring 3 cm in length were treated under conditions where two of the variables were kept constant while the third was varied. As may be seen in Figure 2, the RF plasma a treatment allowed a surface atomic nitrogen concentration of up to 15 % to be reached, with an average N/C ratio of up to 0.3, at 20 W for 250 seconds and with an ammonia pressure of 39,966 Pa (300 mtorr) This represents a significant improvement over previously-reported results following treatment aimed at incorporating nitrogen on ePTFE surfaces, where published data indicates a percentage of nitrogen between 1 and 3 % ^{(26,42,61)} or a N/C ratio between 0.06 and 0.18 ^{(28-43,59)}. A possible explanation for the present results may reside in the cylindrical configuration of the reactor chosen maximizes the efficiency of the treatment ⁽⁵⁹⁾.

The three surface characterization parameters (F/C, N/C and %N) indicate that the surface modifications induced on any given prosthesis are fairly homogcnous : for example, (13 ± 1)% atomic nitrogen concentration on a 7 cm-segment of a 6 mm ID prosthesis (250 s, 39,966 Pa (300 mtorr), 15 W). The degree of homogeneity required for subsequent grafting of the heinocompatible molecule and obtaining of an improved performance of the vascular prostheses can only be properly determined by hemocompatibility tests. However, the observed relative uniformity of surface modifications following ammonia plasma treatment provides a good starting point for the subsequent grafting.

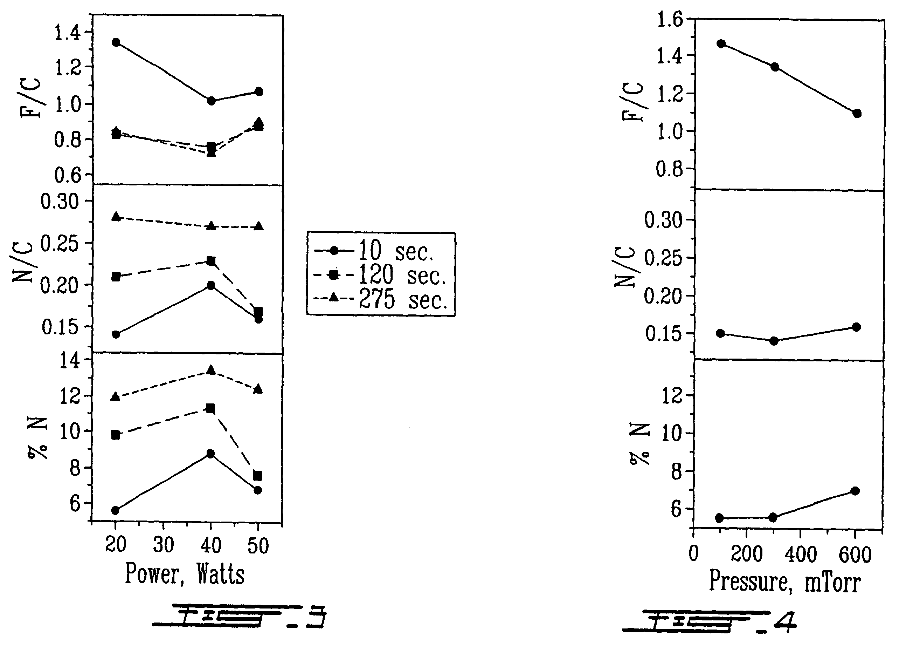

Figure 3 shows the effect on the surface composition of treatments performed by keeping the pressure constant (39966 Pa) (300 mtorr) while varying the power (20, 40 and 50 W) and treatment times (10, 120 and 275 seconds). It appears that the nitrogen concentration reached its highest value for a RF power of 40 W. The N/C ratio measured for the experiments performed for 275 seconds was significantly higher than that measured following treatment for 120 seconds. On the other hand, the F/C ratio was identical for these two treatment times for all RF powers tested. Thus, this data suggests that, despite leading to a higher nitrogen surface concentration, longer treatment times did not promote increased fluorine substitution. This result could mean that different treatment times lead to the formation of different chemical species on the surface.

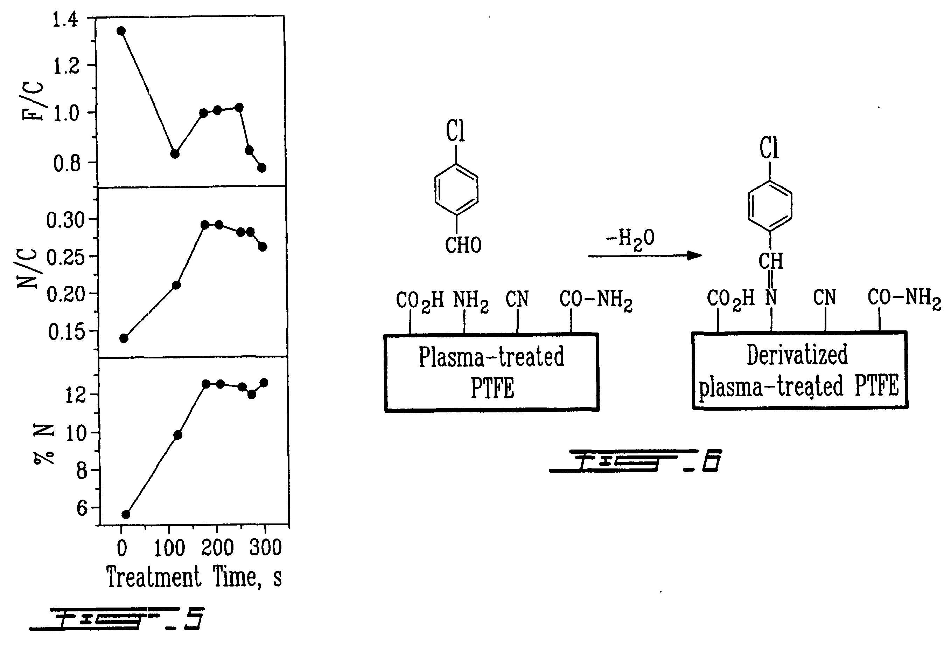

As may be seen in Figure 4, increasing the ammonia pressure from 13,322 to 79,932 Pa (100 to 600 mtorr) (RF power kept at 20W and treatment time at 10s) lead to only a slight increase of the nitrogen content; from lower to higher pressures, this content ranged from 5 to 7%, respectively and the N/C ratio which ranged from 0.14 to 0.16. However, it is clear that the defluorination of the surface is highly pressure-dependent; the F/C ratio decreased from 1.45, for a 13,322 Pa (100 mtorr) pressure, to 1.1 at 79,938 Pa (600 mtorr) Therefore, it is evident that despite a similar nitrogen insertion, the chemical moieties formed on the surface depend upon the pressure within the chamber.

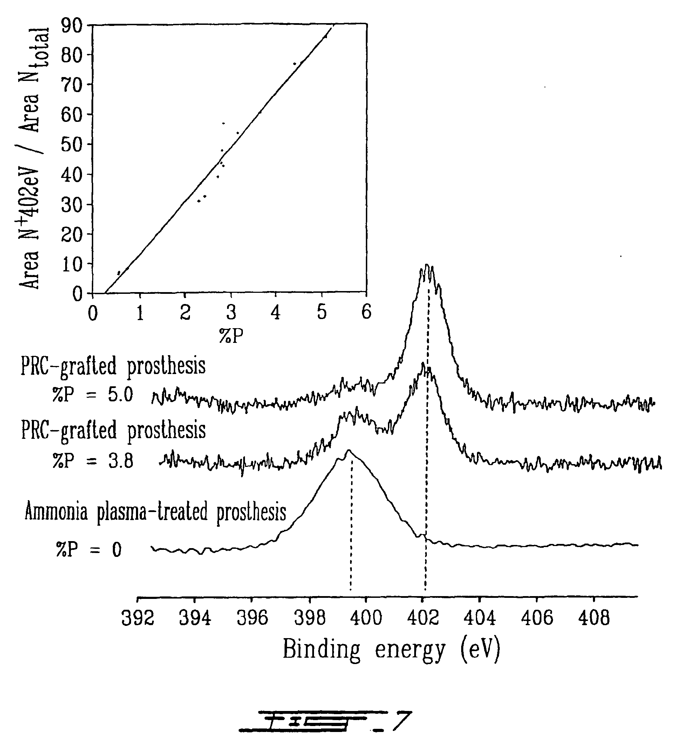

Further experiments were carried out to determine the time required to reach an approximately constant surface concentration of nitrogen. The nitrogen concentration reached a plateau after a treatment time of 160 seconds, as shown by the percentage of nitrogen and the N/C ratio measured on the surface (Figure 5). Defluorination of the surface, despite showing a more complex behavior, appears to follow a similar pattern.

The above results have shown that the pressure, the duration and the RF power of the treatment all have an effect on the chemical composition as seen from the surface characterization parameters given by the survey spectra. In particular, these results raise the possibility of presence of different nitrogen species as a function of the above three treatment parameters.

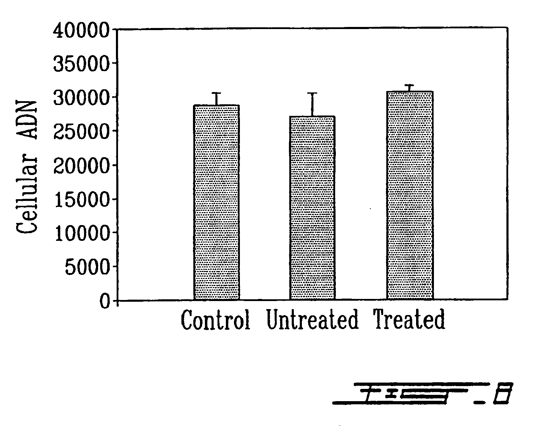

As several nitrogen containing species may be formed upon the ammonia RFGD plasma treatment, we have performed surface derivatization in order to quantify the percentage of the nitrogen present as NH₂ species. The results of the surface derivatization on model surfaces (not shown) demonstrated that chlorobenzaldehyde specifically reacts with the amine moieties as depicted in Figure 6. Therefore, the chlorine XPS signal of the derivatized surface allows the determination of the surface amine concentration as described by d'Agostino et al. ⁽⁶²⁾. Using the surface derivatization technique, it was possible to show that up to 45% of all the nitrogen species grafted onto the surface during the plasma treatment are amine moieties (as shown in Table 1 by % of N₀ present as NH₂). In addition, the data presented in the Table 1 also indicate that the surface amine concentration may be controlled by changing the plasma treatment duration. For example, the surface concentration of NH₂ on the surface goes from 3.6 % for a 50 second treatment duration to 6.0 % when the surface is treated for 250 seconds. However, too long exposures to the plasma environment should be avoided to minimise the occurrence of polytetrafluoroethylene chain scission or formation of double bonds.⁽⁶⁴⁾

**Table 1 : Quantification of amine groups on plasma-treated PTFE film**

| *Plasma treatment* | | | | % of N₀ | Surface concentration |
|---|---|---|---|---|---|
| Duration | F/C | N/C | %N₀ | present as NH₂ | of NH₂ |
| 250s | 0.499 | 0.269 | 14.3 | 42 | 6.0 |
| 100s | 0.626 | 0.229 | 11.9 | 42 | 5.0 |
| 50s | 0.865 | 0.249 | 11.6 | 31 | 3.6 |

One skilled in the art will appreciate that the surface amine groups can be created on the surface by methods different from the one presented here without departing from the spirit of the present invention. For example, amine groups can be created on the surface not only by using an ammonia plasma, but also by using plasmas of N₂H₄ (hydrazinc), ^{(65,66)} aliphatic amincs, ⁽⁶⁷⁾ coating-forming gases such as allylamine, ⁽⁶⁸⁻⁷¹⁾ to name a few non-restrictive possibilities. U.S. Patent 6,159,531, ⁽²⁾ for example, briefly discusses the relative merits of these gases in view of their use as precursors to surface amine groups. Various methods of activating the surface can also be used, other than the continuous capacitively coupled RF plasma used here, are like pulsed plasma, ^{(68,70,73)} inductively coupled RF plasma, ^{(74-76) (77, 78)} and microwave plasma ^{(79, 80) (81,82)}. As an example, one can read U.S. Patent 5,922,161, ⁽⁸³⁾ which teaches the use of such treatments for the partial surface oxidation of polymers.

Furthermore, without departing from the spirit of the present invention, one skilled in the art will appreciate that surface reactive groups other than amine can successfully react with the oxyphosphorodichlorinated derivative form of phosphorylcholine or another so-activated molecule. Hydroxyl and thiol groups ⁽⁸⁴⁻⁸⁷⁾ are two non-restrictive examples of such surface groups. Conversely, different pathways, in addition to the one proposed here, can be followed to obtain an activated form of phosphorylcholine, as one skilled in the art knows. These additional activation methods include, but are not limited to, using reagents such as PCl₅, PCl₃, PBr₅, PBr₃, PI₃, SOCl₂, SOBr₂, COCl₂, COBr₂, (COCl)₂, (COBr)₂ to directly activate phosphorylcholine through the formation of at least a reactive P-halogen bond. ^{(87, 88)}

### Storage in Atmosphere of the ammonia plasma treated prostheses

### Surface atomic concentrations as a function of the storage time in air

The surface atomic concentration of nitrogen on the surface of a treated prosthesis tends to slowly decrease (from 14 % initially to 12 % after 80 days of storage in air or a decrease of N/C from 0.3 to 0.2) and oxygen uptake is also observed (O/C increased from 0.05 to 0.3 after 20 days) ⁽⁴⁵⁾. Therefore, it is preferable to perform the grafting of the molecule shortly after the plasma treatment, preferably within two hours ⁽⁴⁵⁾.

### PRC grafted ePTFE surfaces

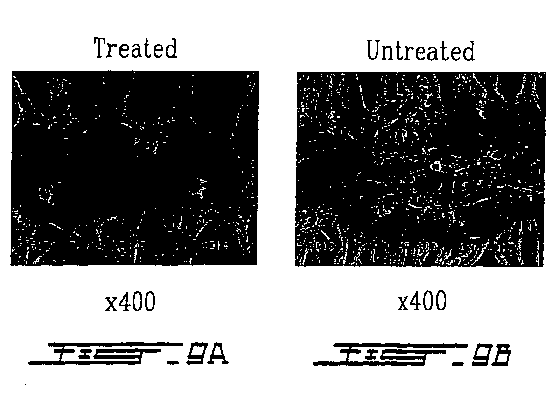

The atomic surface compositions were also determined by XPS spectroscopy. First, the survey spectra clearly showed that there is phosphorus and chlorine atom integration in approximately similar concentrations, as expected from the molecular structure of PRC (chlorine is present as the counterion of the N(CH₃)₃⁺ moiety of PRC). The extent of grafting of the PRC was therefore estimated from the percentage of phosphorus detected by XPS. Moreover, Figure 7 shows that the N 1s High Resolution XPS spectra clearly indicates that there is a good correlation between the trimethylammonium peak at 402 eV (a peak which is not present on an ammonia plasma-treated prosthesis) and the percentage of phosphorus grafted onto the prosthesis. Indeed, there is a linear relation between the extent of PRC grafting (characterized by %P), and the trimethylammonium peak, ⁺N(CH₃)₃ (Figure 7). The C 1s peak (not shown) for each of the three methyl groups from the trimethylammonium moiety, ⁺N(CH₃)₃, followed the same trend and provided further indication of the successful grafting of PRC onto the surface.

The PRC grafting on PTFE films was also confirmed by static SIMS (static Secondary Ion Mass Spectroscopy). This method identifies molecules adsorbed on a surface by recording the mass spectra of atomic or molecular particles which are emitted when a surface is bombarded by energetic primary particles. The secondary ions emitted from the surface result from the fragmentation of the initial surface molecules and are only detected under their ionized state (positive and negative state). The peaks are given in Dalton (molecular weight - gmol⁻¹). Thus, from an analysis of these spectra it is possible to determine the initial structure of the molecules grafted onto the surface.

The spectra of a PRC-grafted PTFE film (not shown) gave the following fragments and confirmed the PRC grafting.

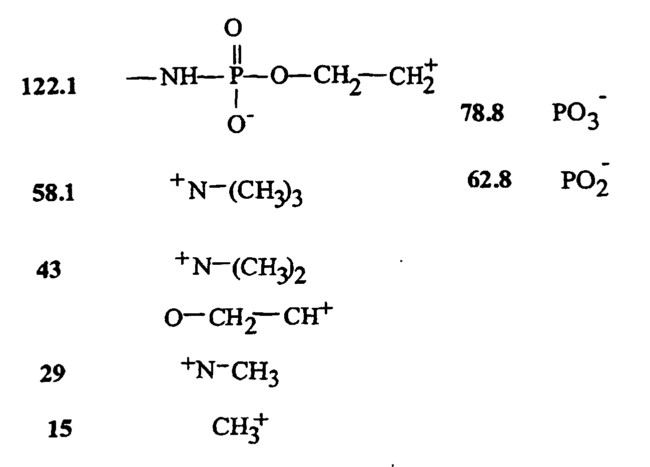

### III. Sterilization of Inner-Surface Modified Vascular Prostheses

For the present method of surface modification to be useful, it is required that sterilization not remove the grafted molecules. A very widely used method for sterilization is autoclaving. At least two possible methods of autoclaving may be contemplated:
1) sterilization under vapor pressure: 120 °C, 15 PSI, 20 min; and
2) dry sterilization: short duration and high temperature (180 °C, 1h) or for a longer duration at a lower temperature (150 °C, 2h).

According to scientific literature, PRC derivatives are stable when subjected to the first sterilization procedure. In addition, ePTFE prostheses are more frequently sterilized in this manner. This method is recognized and used by hospitals.

Sample PRC-grafted films and prostheses were placed in a sterilization bag and sealed. A sterilization marker, a piece of adhesive tape, was placed on the bag; this indicator darkens when sterilization is successful. Following the sterilization procedure, the sample prostheses were analyzed by XPS to determine whether their compositions and the distribution of the phosphorus atoms had been altered. The results are summarized in Table 2 below.

**Table 2: Effect of Sterilization on PRC-Grafted Prostheses**

| Sterilization | Before | After | After 6 months |
|---|---|---|---|
| %P | 5 ± 1 | 4 ± 1 | 3.4 ± 0.6* |

| | | | |
|---|---|---|---|
| * This data was obtained from a 8 cm-segment of a 6 mm ID prosthesis (yielding 60 data points) whereas the other values were estimated from a shorter segment (1 cm, yielding 6 data points), hence the difference in the standard deviations. | | | |

Despite the slight decrease in the percentage of phosphorus, which is taken as a measure of the amount of PRC on the surface because it is otherwise absent, PRC grafting was found to be stable when subjected to sterilization under vapor pressure and the distribution of the grafting on the surface remained homogeneous. The surface concentrations were stable after 6 months and the distribution remained homogeneous (the same was observed for intermediate times of 1 and 2 months for different prostheses). Consequently, the sterilization procedure most practiced by hospitals does not appear to adversely modify the quantity and distribution of the grafted molecules on the PRC-treated vascular prostheses.

### IV. In vitro Testing of Inner-Surface Modified Vascular Prostheses

### A. Non-toxicity of ammonia RFGD plasma treated Prostheses

To verify the non-toxicity of ammonia plasma treated prostheses, we used the diffusion test which determines whether cellular growth is inhibited by the biomaterial by using a coloring agent specific to DNA (Hoechst). The elution test, also known as "extract dilution", was also used; it determines whether there is inhibition of cellular growth by looking for an extract of biomaterial.

### a) Diffusion Test

Human endothelial cells derived from an umbilical cord were cultured in a 24-well plate (1 x 10⁴ cells/well), taking care to pre-treat each well with gelatin before use. The cells were allowed to grow in Medium 199 containing 10% FBS, heparin (90 g/ml), L-Glutamine (2 mM), penicillin G (100 I.U./ml), streptomycin sulfate (100 µg/ml), amphotericin B (250 ng/ml) and ECGS (20 µg/ml). Circular prosthetic samples measuring 7 mm in diameter from both treated and untreated prostheses were placed in the 24 wells. The cultures were incubated for 3 days with 5% CO₂ in a humid atmosphere. Subsequently, the cultures were washed, lysed, treated with Hoechst #33358 and finally transferred to another plate designed for fluorescent studies. Readings were taken with a Bio-Tek FL600 fluorometer, and the quantity of DNA calculated from a calibration curve. At the same time, cells were cultured in wells without prosthetic samples (control).

### b) Elution Test

Two samples, one from a pre-treated prosthesis and the other from an untreated prosthesis, were incubated in M199 for 7 days. Twenty-four hours before the end, endothelial cells were cultured in a 24-well plate. At the seventh day, the cells were cultured in the M199 medium used with the prostheses. (It is important to note here that, in contrast to what is done in the Diffusion Test, only the medium is used; the sample prosthetic devices are set aside following the incubation period.) The endothelial cells are incubated for 3 days under the same conditions as those for the Diffusion Test and analyzed in the same way (fluorometry).

Table 3 shows the results for the Diffusion and Elution Tests on untreated (virgin) prostheses, on ammonia plasma treated prostheses and on control samples. (All experiments were done in duplicate, series A and B.) These results demonstrate that there are no significant variations in the level of cellular proliferation. It may therefore be concluded that ammonia plasma treated ePTFE vascular prostheses are no more toxic than untreated ePTFE vascular prostheses.

**Table 3: Results of in vitro Cellular Cultures Analyzed by Fluorometry**

| **Test** | **Untreated Prostheses** | **Ammonia Plasma treated Prostheses** | **Control** |
|---|---|---|---|
| | (*10⁴) | (*10⁴) | (*10⁴) |
| **Diffusion** | | | |
| Series A | 54 ± 1 | 52 ± 2 | |
| Series B | 43.7 ± 0.5 | 43.1 ± 0.5 | 40 ± 1 |

| **Elution** | | | |
|---|---|---|---|
| Series A | 32.9 ± 0.4 | 33 ± 1 | |
| Series B | 28 ± 1 | 32 ± 1 | 31 ± 2 |

### Cytotoxicity of the PRC-grafted vascular prostheses

As discussed above, toxicity tests performed on prostheses following plasma treatment but prior to the anchoring of the molecules revealed that the plasma-treated prostheses were non-toxic, or at least no more toxic than virgin prostheses. The next logical step was to determine whether prostheses which had been completely treated by the process of the present invention were also non-toxic to cells, and particularly to endothelial cells which naturally line the internal surface of blood vessels. Endothelial cells are currently used to verify the toxicity of certain products as well as to check the efficiency of sterilization. In cytotoxicity testing, the prostheses are not in direct contact with endothelial cells and the incubation period is 3 days at 37 °C. Cellular growth is determined by the fluorescence of DNA strands using specific markers.

Testing was repeated several times in order to obtain the best statistical average for the cell culture results. To gain a better appreciation of the effect of PRC grafting on the cells, tests were also performed on untreated prostheses as well as with "control" cell cultures.

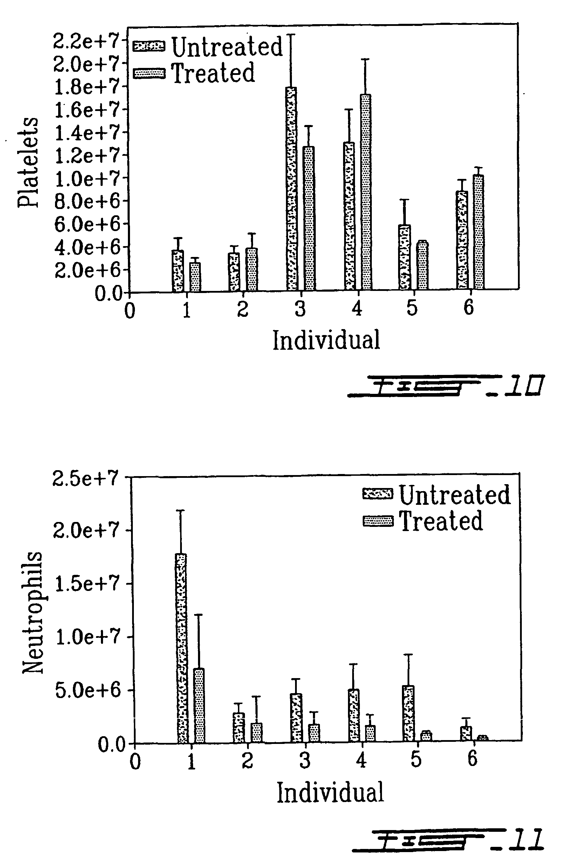

The histogram in Figure 8 clearly shows that treated prostheses have no more effect on cell growth than do untreated prostheses and neither of them have a significant effect on the growth of the cells. Hence, the prostheses treated with the process of the invention are non-cytotoxic and in fact seem to slightly favor endothelial cell growth. These findings are promising given that endothelial cells must line the internal surfaces of the prostheses.

### B) Blood Platelets Adhesion

### Adhesion and Aggregation of Blood Platelets

The adhesion and aggregation of blood platelets on prosthetic devices has a direct effect on hemocompatibility, since the platelets are involved in the coagulation cascade. Indeed, the adherence of platelets on the internal surfaces of prostheses may prevent the adherence of endothelial cells (the natural lining of blood vessels), and may result in thrombosis if the accumulation is too important.

*In vitro* studies were performed several times in the following manner. Following the centrifugation of human blood, the platelet-rich plasma was extracted and placed on prostheses for one hour at 37 °C. The prostheses were then treated with different solutions before analyses by SEM.

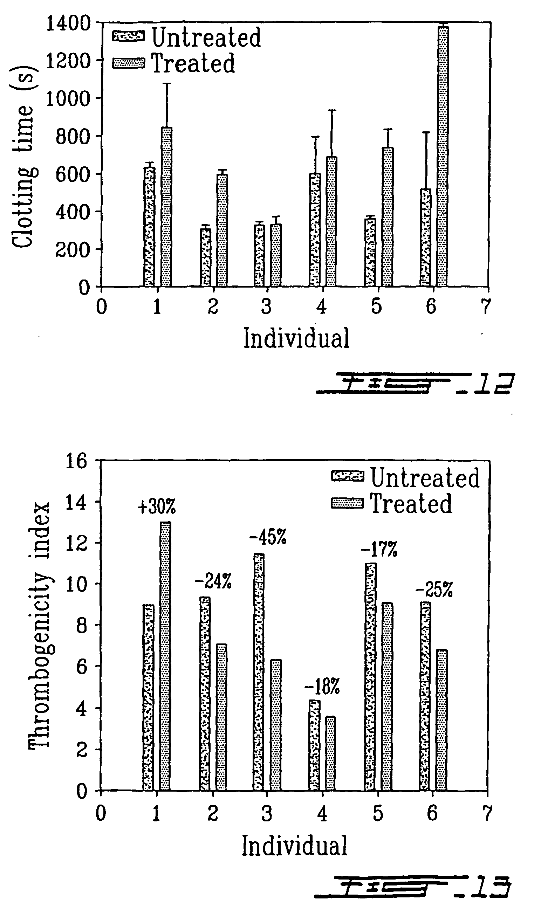

Figure 9 shows SEM photographs of treated and untreated prosthetic devices. A comparison of the two photographs reveals that the untreated prosthesis has a significantly greater accumulation and activation of platelets than the prosthesis that had been treated with the process of the invention. Hence, the treated prosthesis appears to prevent much more effectively the accumulation of blood platelets.

### Quantification of Blood Platelets

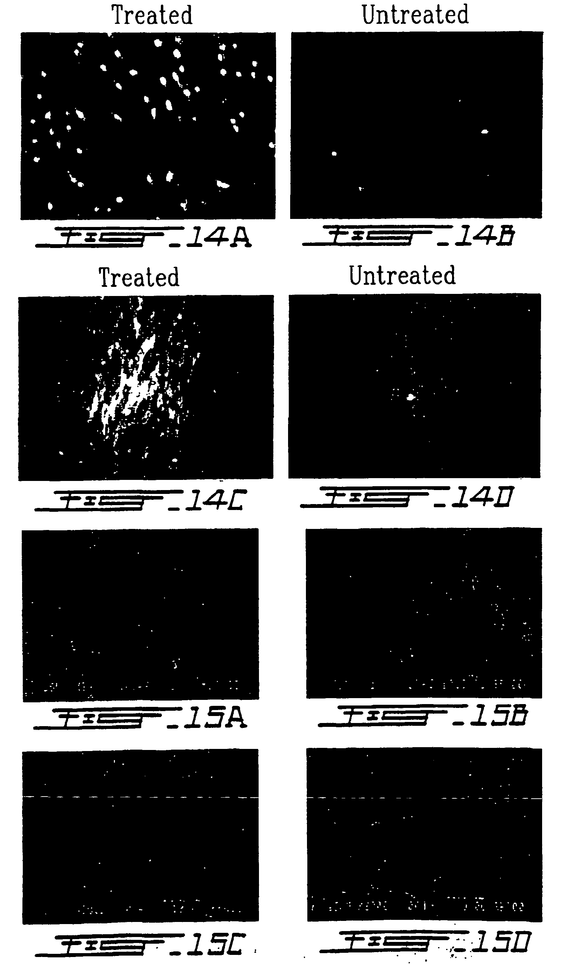

After centrifugation of human blood, the platelet-rich plasma (5 * 10⁴ platelets /µl count by a hemocytometer) was extracted, radiolabelled with ⁵¹Cr and placed on prostheses for two hours at 37 °C. The prostheses were then washed three times with sterile PBS before analyses and the amount of platelet uptake was evaluated by a gamma scintillation counter adjusted to 270-370keV. This test was performed 3 times with human blood from six donors (Figure 10).

In all cases, no significant difference in the adherent platelets was noticed for untreated and treated prostheses.

### C) Neutrophils

Neutrophils are characteristic of the inflammatory response of an individual to a foreign material.

After centrifugation of human blood, the leukocyte-rich suspension was extracted, radiolabelled with ¹¹¹In and placed on prostheses for two hours at 37 °C. The prostheses were then analyzed and the adherent neutrophils were evaluated by a gamma scintillation counter adjusted to 260-480keV. This test was performed three times with blood from six different donors (Figure 11).

For every individual, there were fewer adherent neutrophils on the treated prosthesis than on the untreated prosthesis. Thus, it appears that the inflammatory response was reduced by the PRC grafted on the prosthesis.

### D) Clotting time

Platelet adhesion to biomaterials is often used as an indication of blood compatibility, but a more clinically relevant issue is whether the adherent platelets are able to promote clot formation. Indeed, clotting events require the presence of other blood elements such as fibrinogen to catalyze the clotting cascade. Longer clotting times are taken as indicative of a better thrombogenic resistance of a prosthesis in contact with blood.

Normal human blood was collected into a citrate anticoagulant tube and a 1-ml sample was deposited onto the surface of the prosthesis. The clotting time was determined visually: the blood was deemed to have clotted when there was no longer a movement of the blood in response to a tilting motion of the sample prosthesis. This test was performed three times with blood from six human donors, on untreated and treated (PRC-grafted) prostheses (Figure 12).

As observed in Figure 12, clotting times were significantly longer on PRC-grafted prostheses. Thus, it appears that the treated prosthesis is less thrombogenic when compared to the untreated prosthesis.

### E) Thromboelastography

Another important measure of the thrombogenic potential of a material is the evolution of the elastic properties of whole blood clots that form on the surface of the material. For a given material in contact with blood, the thrombogenicity index takes into account the coagulation time as well as the elasticity of the blood clot. Surfaces with good blood compatibility should exhibit low thrombogenecity indexes, as measured by a thromboelastograph. This technique uses a steel piston placed in a cuvette having a 1-mm clearance between the piston and the inner surface of the cuvette. Blood is placed inside the cuvette. As coagulation proceeds, the elasticity of the forming blood clot varies and these changes are detected by the torsion wire to which the piston is suspended. This is converted to an electric signal which is sent to a chart recorder. The resulting trace is called the thromboelastogram (TEG), which records the elasticity of the formed blood clot. This test was performed using the blood of six human donors, on samples of untreated and treated prostheses (Figure 13).

Comparison of the index of thrombogenic potential for treated and untreated prostheses showed a lower thrombogenic potential for the PRC-grafted prostheses. Values ranged from 17 to 45% lower, except for one individual where the value was 30 % higher. Thus, the treated prostheses improved the non-thrombogenic potential of the initial material.

### F) Fibroblast Cultures

The development of an endothelium, which is the natural hemocompatible surface, on the surface of prostheses would render them more hemocompatible. Fibroblasts, which are cells capable of differentiation, are commonly used for *in vitro* hemocompatibility tests. By testing the adsorption of fibroblasts, one can measure the ability of a material to act as a viable substrate for the adsorption and spreading of cells. The adsorption and spreading of cells represent conditions that are necessary for the development of the endothelium.

Fibroblasts were placed in direct contact with the prostheses in order to observe the ability of cells to develop on different materials. The cells were allowed to grow for 7 days at 37 °C. The fibroblasts were then observed through a microscope using two coloring agents: the Hoëscht reagent marked the nucleus (allowing the cells to be counted) and rhodamine dyed the actin filaments in the cytoplasm (allowing the shape of the cells to be determined).

As shown in Figure 14, the development of fibroblasts on the surface of the prostheses treated by the process of the present invention was greatly enhanced in comparison to the untreated prosthesis: the number of nuclei is greater, and the shape of the fibroblasts is more elongated, which shows that the cells adhere to and develop on the surface.

### G) Endothelial Cell Cultures

Endothelial cells are the cells that naturally line the internal surface of blood vessels; they therefore constitute the ideal hemocompatible surface. Because endothelial cells are known to be more fragile and sensitive than fibroblasts, this test is more severe. Endothelial cells were cultured following the same protocol as that used for the fibroblasts. The incubation period was 3 days at 37 °C. The development of endothelial cells was greatly enhanced on the prosthesis treated by the process of this invention than on the untreated one. Figure 14 shows the optical microscope image obtained after using the Hoëscht reagent to mark the nucleus of the endothelial cells. The number of cell nucleus is clearly more important on the treated prosthesis, as is the spreading of the cells, as observed by SEM (Figure 14).

### V. In vivo Testing of Inner-Surface Modified Vascular Prostheses

The *in vivo* response of the ANBIOPA™-treated prostheses was tested in a canine model. The implantation period was one month and six dogs received a prosthesis: three received an untreated Gore-Tex™ ePTFE prosthesis while the three others received an ANBIOPA™-treated prosthesis.

### Surgery

(The following procedure, while described in relation to a single dog, was used on all six dogs that participated in the trial.)

After a 24-hour fast, a conditioned mongrel dog having a weight of between 20 and 25 kg was pre-anaesthetized by intra-veinous administration of atropin sulfate (0.05mg/kg) and acepromazin maleate (0.1mg/kg). The dog's abdomen was shaved and disinfected by an application of Hibitane and Proviodine. The dog was then placed on a surgical table in decubitus position, anaesthetized by intra-veinous administration of Penthothal (10mg/kg) and then ventilated with a mix of air and isofuran to maintain anaesthesia. After abdominal incision, the sub-renal aorta was localized and cleared of surrounding structures before the collateral arteries were tied. Prior to clamping, heparin (0.5mg/kg) was injected as an anti-coagulant. The aorta was clamped upstream and downstream of a 5 cm segment in an aorto-aortic position and this segment was replaced with a 6mm diameter vascular prosthesis (untreated commercially available prothesis or ANBIOPA™-treated prosthesis) of the same length by termino-terminal anastomosis using a Surgilene™ monofilament. The dog was stitched and butorphanol tartrate (0.2mg/kg) was subcutaneously injected as an analgesic for three days after surgery. Following surgery, the dog was fed with a standard diet until explantation of the prosthesis one (1) month later.

### Image analysis

SEM observation on the explanted prostheses shows clear differences between the untreated commercially available prostheses and the ANBIOPA™-treated prostheses (Figure 15). At low magnification, the surface of an ANBIOPA™-treated prosthesis remains uniform with a smooth surface and is almost deposit free (Fig. 15 a). Further observations at higher magnification reveal a coating consisting of several layers of red blood cells (Fig. 15 c). The lack of activated platelets and fibrin clot on an ANBIOPA™-treated prosthesis surface suggests an anti-thrombogenic property. On the other hand, an untreated commercially available prosthesis shows important deposits on the surface at low magnification (Fig 15 b) with deposits mainly composed of a network of activated platelets and red blood cells trapped in a fibrin clot (Fig. 15 d), thus providing evidence of thrombus formation.

### LIST OF REFERENCES

1. R. Guidoin, N. Chakfé, S. Maurel, T. How, M. Batt, M. Marois and C. Gosselin, *Biomaterials* **14**, 678 (1993).
2. B.D. Ratner, A. Chilkoti and G. P. Lopez, Plasma deposition and treatment for biomedical applications, in *Plasma Deposition, Treatment, and Etching of Polymers,* R. D'Agotino, ed., Academic Press, New York, (1990), p. 463.
3. B.D. Ratner, *J. Biomed. Mat. Res.* **27**, 837 (1993).
4. B. Haimovich; A. Freeman; R. Greco, Thromboresistant surface treatment for biomaterials, U.S. Pat. 5,578,073 (1996).
5. P.D. Drumheller, Materials and method for the immobilization of bioactive species onto polymeric subtrates, U.S. Pat. 5,874,165 (1999).
6. J.R. Keogh, Ionic attachement of biomolecules with a guanidine moiety to medical devices surfaces, U.S. Pat. 5,928,916 (1999).
7. J.M. McHaney and C.E. Banas, Carbon containing vascular graft and method of making same, U.S. Pat. 5,827,327 (1998).
8. J.R. Keogh, Method for a covalent attachment of biomolecules to surfaces of medical devices, U.S. Pat. 6,033,719 (2000).
9. J.R. Keogh, Periodate oxidative method for attachment and crosslinking of biomolecules to medical device surfaces, U.S. Pat. 6,017,741 (2000).
10. J.R. Keogh, Method for attachment of biomolecules to medical devices surfaces, U.S. Pat. 5,925,552 (1999).
11. J.R. Keogh, Oxidative method for attachment of glycoproteins or glycopeptides to surfaces of medical devices, U.S. Pat. 5,891,506 (1999).
12. J.R. Keogh, Oxidative method for attachment of glycoproteins to surfaces of medical devices, U.S. Pat. 5,728,420 (1998).
13. P.T. Cahalan, M. Verhoeven, M. Hendricks, L. Cahalan, Biocompatibility of solid surfaces, U.S. Pat. 5,702,818 (1997).
14. P. E. Guire, Biocompatible device with covalently bonded biocompatible agent, U.S. Pat. 5,263,992 (1993).
15. D.G. Castner, P. Favia and B.D. Ratner, Deposition of fluorocarbon films by remote RF glow discharges, in *Surface Modification of Polymeric Biomaterials,* B.D. Ratner and D.G. Castner, eds., Plenum Press, New York (1997), p. 45.
16. B. D. Ratner, D. Leach-Scampavia and D. G. Castner, *Biomaterials* **14**, 148 (1993).
17. A. S. Hoffman, A. M. Garfinkle, B. D. Ratner and S. R. Hanson, Plasma gas discharge treatment for improving the compatibility ofbiomaterials, U.S. Pat. 5,034,265 (1991).
18. T. A. Horbett, B. D. Ratner, J. A. Chinn and Y. Haque, Radio frequency plasma deposited polymers that enhance cell growth, U.S. Pat. 4,919,659 (1990).
19. A. S. Hoffman, A. Garfinkle, B. D. Ratner and S. R. Hanson, Plasma gas discharge treatment for improving the biocompatibility of biomaterials, U.S. Pat. 4,656,083 (1987).
20. M. Garfinkle, A. S. Hoffmann, B. D. Ratner, L. O. Reynolds, S. R. Hanson, *Trans. Am. Soc. Artif. Intern. Organs* **30**, 432 (1984).
21. J. C. Lin and S. L. Cooper, *J. Appl. Polym. Sci.: Appl. Polym. Symp.* **54**, 157 (1994).
22. P. V. Narayanan, *J. Biomater. Sci. Polymer Edn,* **6,** 181 (1994).
23. Y. Matsuzawa and H. Yasuda, *J. Appl. Polym. Sci.* **38,** 65 (1984).
24. P. Favia, V. H. Perez-Luna, T. Boland, D.G. Castner and B.D. Ratner, *Plasmas & Polymers* **1**, 299, (1996).
25. Y. Babukutty, M. Kodama, H. Nomiyama, M. Kogoma and S. Okazaki, Preparation of acrylamide coated PVC tube by APG treatment and its characterization, in *Advanced Biomaterials in Biomedical Engineering and Drug Delivery Systems,* N. Ogata, S. W. Kim, J. Feijen and T. Okano, eds., Springer-Verlag, Tokyo (1996), p. 217.
26. A. Dekker, K. Reitsma, T. Beugeling, A. Bantjes, J. Feijen and W. G. van Aken, *Biomaterials* **12**, 130 (1991).
27. T. A. Giroux and S.L. Cooper, *J. Colloid Interface Sci.* **146,** 179 (1991).
28. N. Inagaki, S. Tasaka and H. Kawai, *J. Adhesion Sci. Technol.* **3,** 637 (1989).
29. Y. Nakayama, T. Takahagi, F. Soeda, S. Nagaoka, J. Suzuki and A. Ishitani, *J. Polym. Sci., Part A: Polym. Chem.* **26,** 559 (1988).
30. J. R. Hollahan, B. B. Stafford, R. D. Falb and S. T. Payne, *J. Appl. Polymer Sci.* **13**, 807 (1969).
31. R. E. Marchant, Anticoagulant plasma polymer-modified substrate, U.S. Pat. 5,455,040 (1995).
32. H. K. Yasuda, Method of making a biocompatible prosthesis, U.S. Pat. 4,994,298 (1991).
33. T. Karwoski and Y. Matsuzawa, Glow discharge process for producing implantable devices, U.S. Pat. 4,632,842 (1986).
34. P. V. Narayanan, Radiofrequency plasma biocompatibility treatment of inside of medical tubing and the like, U.S. Pat. 5,244,654 (1993).
35. P. V. Trescony, P. Cahalan and K. Keeney, Vascular prosthesis and method, U.S. Pat. 5,246,451 (1993).
36. P. V. Narayanan, S. M. Rowland and K. D. Stanley, Treatment of metallic surfaces using radiofrequency plasma deposition and chemical attachment of bioactive agents, U.S. Pat. 5,336,518 (1994).
37. P. V. Narayanan, Radiofrequency plasma biocompatibility treatement of inside surfaces, U.S. Pat. 5,486,357 (1996).
38. F. Schué, G. Clarotti, J. Sledz, A. Mas, K. E. Geckeler, W. Göpel, A. Orsetti, *Makromol. Chem, Macromol. Symp.* **73,** 217 (1993).
39. R. Sipehia, *Biomat., Art. Cells & Immob. Biotech.* **21,** 647 (1993).
40. U. König, A. Augsburg, F. Simon, D. Lunkwitz, G. Hermel, C. Wemer, M. Müller, H. J. Jacobash, *ACS Polymer Preprints.* **38,** 1079 (1997).
41. F. Simon, G. Hermel, D. Lunkwitz, C. Wemer, K. Eichhorn and H. J. Jacobash, *Macromol. Symp.* **103**, 243 (1996).
42. R. Sipehia, G. Martucci, M. Barbarosie and C. Wu, *Biomat., Art. Cells & Immob. Biotech.* **21**, 455 (1993).
43. D. Ratner, Biomaterials science: an interdisciplinary endeavor, in *Biomaterials science : an introduction to materials in medicine,* B. D. Ratner, A. S. Hoffman, F. J. Schoen, J. E. Lemons, eds., Academic Press, San Diego, (1996), p. 1.
44. D. Mantovani, M. Castonguay, J.F. Pageau, M. Fiset and G. Laroche. *Mater. Res. Soc. Preprints,* **544**, 21 (1998).
45. D. Mantovani, M. Castonguay, J.F. Pageau, M. Fiset and G. Laroche. *Plasmas and Polymers,* **4**, 207 (1999).
46. J. A. Hayward and D. Chapman, *Biomaterials,* **5**, 135 (1984).
47. R. F. A. Zwall , P. Comfurius, L. M. M. van Deenen, *Nature,* **268**, 358 (1977).
48. R. F. A. Zwall , H. C. Hember, *Haemostasis,* **11,** 12 (1982).
49. R. F. A. Zwall and E. M. Bevers, *Subcellular Biochemistry,* **9**, 163 (1983).
50. Y. P. Yianni, Biocompatible surfaces based upon biomembrane mimicry, in *Structural and dynamic properties of lipids and membranes,* eds., P J Quinn & R J Cherry, Portland, Press Research Monograph, 1992, p. 187-216.
51. A. A. Durrani, J. A. Hayward and D. Chapman, *Biomaterials,* **7,** 121 (1986).
52. Y. P. Yianni, S. R. Hanson, A.B. Lumsden, *Journal of Surgical Research,* **77,** 119 (1998).
53. K. Ishihara, R. Aragaki, A. Watenabe, N. Nakabayashi, *J. Biomed. Mater. Res*., **24**, 1069 (1990).
54. K. Ishihara, N. Nakabayashi, K. Fukumoto, J. Aoki, *Biomaterials,* **13**, 145 (1992).
55. K. Ishishara, H. Oshida, Y. Endo, T. Ueda, A. Watanabe, N. Nakabayashi, *J. Biomed. Mater. Res.,* **26,** 1543 (1992).
56. K. Ishihara, H. Hanuyada, N. Nakabayashi, *Biomaterials*,**16**, 873 (1995).
57. Y. Iwasaki, K. Ishihara, N. Nakabayashi, G. Khang, J. H. .Jeon, J. W. Lee, H.B. Lee, *JBiomater Sci Polym Edn,* **9***,* 801 (1998).
58. A. E. Gal, F. J. Fash, *Lipids,* **12,** 314 (1976).
59. J.C. Lin and S.L. Cooper, *J. Appl. Polymer Sci. : Appl. Polymer Symp*. **54,** 157 (1994).
60. F. Fracassi, Architecture of RF plasma reactors, in *Plasma Processing of Polymers,* R. D'Agostino, P. Favia, F. Fracassi, eds., NATO-ASI Series E : Applied Science - Vol. 346, Kluwer Academic Publishers (1997), p. 47.
61. M. E. Ryan and J.P.S. Badyal, *Macromolecules* **28**, 1377 (1995).
62. P. Favia, M. V. Stendardo and R. d'Agostino, *Plasmas and Polymers* **1**, 91 (1996).
63. S. F. Popescu, M. Castonguay, C. Doillon, R. C. Gaudreault, G. Laroche. *Society for Biomaterials 25*^{*th*} *Annual Conference,* Proceeding vol. XXII, 574, April 28 - May 2, 1999, Rhode Island, USA.
64. P. Chevallier, M. Castonguay, S. Turgeon, D. Mantovani, J.C. Wittmann, G. Laroche, , submitted for publication at *J. Phys. Chem.*
65. A. J. Martinez, S. Manolache, V. Gonzalez, R. A. Young, F. Denes, *J*. *Biomater. Sci., Polym. Ed*., **11**, 415 (2000).
66. X. Zhang, R. A. Young, *Mater. Res. Soc. Symp. Proc.,* **586,** 157 (2000).
67. J. G. A. Terlingen, L. M. Brenneisen, H. T. J. Super, A. P. Pijpers, A. S. Hoffman, J. Feijenand, *J. Biomater. Sci., Polym. Ed.,* **4,** 165 (1993)
68. W. R. Gombotz, A. S. Hoffman, *J. Appl. Polym. Sci. : Appl. polym. Symp.,* 42, 285 (1988).
69. H. J. Griesser, *Mater. Forum,* **14**, 192 (1990).
70. C. L. Rinsch, X. Chen, V. Panchalingam, R. E. Eberhart, J-H. Wang, R. B. Timmons, *Langmuir,* **12**, 2995 (1996).
71. J. G. Calderon, A. Harsch, G. W. Gross, R. B. Timmons, *J. Biomed. Mater. Res.,* **42**, 597 (1998).
72. M. H. Dang; P. Chiu, Coating having biological activity and medical implant having surface carrying the same and method, U.S. Pat. 6,159,531 (2000).
73. J. G. Calderon, R. B. Timmons, *Macromolecules,* **31**, 3216 (1998).
74. P. Colpo, R. Ernst, J.-P. *Keradec, Plasma Sources Sci. Technol.,* **8**, 587 (1999).
75. M. Desilets, B. Davies, G. Soucy, P. Proulx, *Can. J. Chem. Eng.,* **76**, 707 (1998).
76. J. T. Gudmundsson, A. M. Marakhtanov, K. K. Patel, V. P. Gopinath, M. A. Lieberman, *J. Phys. D: Appl. Phys.,* **33**, 1323 (2000).
77. R. Gasparik, S. Ihara, C. Yamabe, S. Satoh, *Jpn. J. Appl. Phys., Part 1,* **39**, 306 (2000).
78. J. Lei, M. Shi, J. Zhang, *Eur. Polym. J.,* **36**, 1277 (2000).
79. R. Foerch, J. Izawa, N. S. Mc Intyre, *J. Appl. Polym. Sci. : Appl. Polym. Symp.,.***46***, 415* (1990).
80. A. E. Lefohn, N. M. Mackie, E. R. Fisher, *Plasmas and Polymers,* **3,** 197 (1998).
81. S. I. Kim, S. W. Lee, Y. H. Park, M. Ree, *Mol. Cryst. Liq. Cryst. Sci. Technol., Sect. A,* **349,** 275 (2000).
82. J. M. Nedelec, J. Grimblot, S. Turrell, M. Bouazaoui, *J. Phys. Chem. B,* **104**, 926 (2000).
83. D. Y. Wu; S. Li; G. W. Stanislaw, Surface treatment of polymers, U.S. Pat. 5,922,161 (1999).
84. Based on the international conference on phosphorus chemistry at Duke University, Durham, North Carolina (1981), Phosphorus chemistry Proceeding of the 1981 international conference, American Chemical Society, Eds. Library of congress CIP , Data, Vol. 171.
85. R. Neidlen, S. Buseck, *Helvetica Chimica Acta,* **75,** 2520 (1992).
86. R. Neidlen, P. Greulich, *Helvetica Chimica Acta,* **75,** 2545 (1992).
87. Theilheimer, Synthetic Methods of Organic Chemistry, Finch, Theilheimer, Nutley, Tozer, Eds Karger, Interscience publishers, Basel - München - Paris - London - New-York - New Dehli - Singapore - Tokyo - Sydney, 1946-1998.
88. A. I. Vogel, Vogel's Textbook of Practical Organic Chemistry, including qualitative organic analysis, 4th ed. / rev. by the following members of the School of Chemistry, Thames (formerly Woolwich) - polytechnic, B. S. Furniss ...[et al.], London; New York: Longman, 1978.

## Claims

1. A process for modifying the surface properties of a material suitable for contact with a living tissue comprising:
-- Exposing the surface of the material to plasma treatment conditions in order to create reactive groups on said surface;
-- Activating a molecule with phosphorylchloride (POCl3) to produce a reactive molecular species capable of forming covalent bonds with the reactive groups created on the surface of the material; and
-- Contacting the reactive molecular species with the reactive groups created on the surface of the material to form covalent bonds.

2. A process as defined in claim 1, wherein the resulting surface of the material resulting is compatible with living tissue.

3. A process as defined in claim 2, wherein said tissue is blood tissue.

4. A process as defined in claim 1, wherein :
-- said plasma is ammonia Radio Frequency (RF) plasma and said reactive groups are amine groups;
-- said molecule is selected from the group consisting of choline, heparin, and other molecules known to those skilled in the art for their hemocompatibility, and said reactive molecular species is the oxyphosphorodichlorinated derivative of said molecule; and
-- said covalent bonds are phosphoamide-type covalent bonds.

5. A process as defined in claim 4, wherein said plasma treatment conditions consist of the application of a RF power of between 5 watts to 500 watts for a time of 10 seconds to 30 minutes at a pressure of 6,661 Pa (50 mtorr) to 666,1 Pa (5 torr).

6. A process as defined in claim 5, wherein said plasma treatment conditions consist of the application of a RF power of 20 watts for a time of 250 seconds at a pressure of 39,966 Pa (300 mtorr).

7. A process as defined in claim 5, wherein said plasma treatment conditions consist of the application of a RF power of 15 watts for a time of 100 seconds at a pressure of 33,305 Pa (250 mtorr).

8. A process as defined in claim 6 or 7, wherein contacting the reactive molecular species with the reactive bonds is performed within 2 hours of exposing the surface of the material to plasma treatment.

9. A process as defined in any one of claims 1 to 8, wherein said surface of said material suitable for contact with a living tissue is material implantable in an animal's body.

10. A process as defined in claim 9, wherein said surface of said material implantable in an animal's body is selected from the group consisting of:
Microporous expanded polytetrafluoroethylene (ePTFE), polytetrafluoroethylene (PTFE), polyvinylchloride (PVC), polyethylenes, polyesters (such as polyethylene terephtalate - PET), polypropylenes, polyurethanes, polycarbonates, silicones, PVDF, and polymer-coated materials, such as metals and ceramics for example.

11. A process as defined in claim 10, wherein said surface of said material implantable in an animal's body is ePTFE.

12. A process as defined in claim 11, wherein said surface is the internal surface of a vascular prosthesis treated with ePTFE.

13. A process as defined in claim 12, wherein said vascular prosthesis has an inner diameter of 1 to 30 mm.

14. A material implantable in an animal's body produced by the process of any one of claims 1 to 13.

15. A vascular prosthesis produced by the process of claim 12 or 13.

16. A device comprising a material as defined in claim 14.

17. A device as defined in claim 16, wherein said device is to be used in contact with an animal's tissue.

18. A device as defined in claim 17, wherein said tissue is blood tissue.

19. A device as defined in claim 17 or 18, wherein said device is selected from the group consisting of:
An implant, a prosthesis, an artificial organ, a stent, a cardiac valve, an apparatus contacting blood during an extra-corporal blood circulation, an apparatus contacting blood during a dialysis treatment or any other material with surfaces coming in contact with blood.

20. Use of a material as defined in claim 14 in the manufacture of a device for preventing thrombosis.

21. A material produced by the process of any of claims 1 to 13 suitable for contact with a living tissue **characterized in** having a surface that is resistant to neutrophil adhesion, platelet adhesion and activation but that enhances the development of fibroblasts and endothelial cells.

22. A material as defined in claim 21 which is plasma-treated ePTFE with covalently grafted phosphorylcholine (PRC).

23. A device comprising a material as defined in claim 21 or 22.

24. A device as defined in claim 23, being selected from the group consisting of:
An implant, a prosthesis, an artificial organ, a stent, a cardiac valve, an apparatus contacting blood during an extra-corporal blood circulation, an apparatus contacting blood during a dialysis treatment or any other material with surfaces coming in contact with blood.

25. Use of a material as defined in claim 21 or 22 in the manufacture of a device having surface treated for preventing thrombosis.

## Patentansprüche

1. Verfahren, um die Oberflächeneigenschaften eines Materials, das für den Kontakt mit einem lebenden Gewebe geeignet ist, zu modifizieren, aufweisend:
- Aussetzen der Oberfläche des Materials gegenüber Bedingungen der Plasmabehandlung, um auf der Oberfläche reaktive Gruppen zu schaffen;
- Aktivieren eines Moleküls mit Phosphorylchlorid (POCl₃), um cine reaktive molekulare Spezies herzustellen, die in der Lage ist mit den auf der Oberfläche des Materials geschaffenen reaktiven Gruppen kovalente Bindungen auszubilden, und
- In-Kontakt-Bringen der reaktiven molekularen Spezies mit den auf der Oberfläche des Materials geschaffenen reaktiven Gruppen, um kovalente Bindungen auszubilden.

2. Verfahren nach Anspruch 1, wobei die resultierende Oberfläche des resultierenden Materials mit lebendem Gewebe kompatibel ist.

3. Verfahren nach Anspruch 2, wobei das Gewebe Blutgewebe ist.

4. Verfahren nach Anspruch 1, wobei:
- das Plasma ein Ammoniak-Hochfrequenz(HF)-Plasma ist und die reaktiven Gruppen Amingruppen sind;
- das Molekül ausgewählt ist aus der Gruppe bestehend aus Cholin, Heparin und anderen Molckülen, die dem Fachmann für deren Hamokompaubilität bekannt sind, und die reaktive molekulare Spezies das oxyphosphorodichlorinierte Derivat des Moleküls ist, und
- die kovalenten Bindungen kovalente Bindungen vom Phosphoamidtyp sind.

5. Verfahren nach Anspruch 4, wobei die Bedingungen der Plasmabehandlung aus dem Anlegen einer HF-Leistung von 5 Watt bis 500 Watt für eine Dauer von 10 Sekunden bis 30 Minuten bei einem Druck von 6,661 Pa (50 mtorr) bis 666,1 Pa (5 torr) bestehen.

6. Verfahren nach Anspruch 5, wobei die Bedingungen der Plasmabehandlung aus dem Anlegen einer HF-Leistung von 20 Watt für eine Dauer von 250 Sekunden bei einem Druck von 39,966 pa (300 mtorr) bestehen.

7. Verfahren nach Anspruch 5, wobei die Bedingungen der Plasmabehandlung aus dem Anlegen einer HF-Leistung von 15 Watt für eine Dauer von 100 Sekunden bei einem Druck von 33,305 Pa (250 mtorr) bestehen.

8. Verfahren nach Anspruch 6 oder 7, wobei das In-Kontakt-Bringen der reaktiven molekularen Spezies mit den reaktiven Bindungen innerhalb von zwei Stunden des Aussetzens der Oberfläche des Materials gegenüber einer Plasmabehandlung durchgeführt wird.

9. Verfahren nach einem der Ansprüche 1 bis 8, wobei die Oberfläche des Materials, das für den Kontakt mit einem lebenden Gewebe geeignet ist, ein Material ist, das in einen Körper eines Lebewesens implantierbar ist.

10. Verfahren nach Anspruch 9, wobei die Oberfläche des Materials, das in einen Körper eines Lebewesens implantierlaar ist, ausgewählt ist aus der Gruppe bestehend aus:
mikroporösem expandiertem Polytetrafluoroethylen (ePTFE), Polytetrafluoroethylen (PTFE), Polyvinylchlorid (PVC), Polyethylenen, Polyestern (wie bspw.
Polyethylenterephthalat - PET), Polypropylenen, Polyurethanen, Polycarbonaten, Silikonen, PVDF und polymerbeschichteten Materialien, wie bspw. Metallen und Keramiken.

11. Verfahren nach Anspruch 10, wobei die Oberfläche des Materials, das in einen Körper eines Lebewesens implantierbar ist, ePTFE ist.

12. Verfahren nach Anspruch 11, wobei die Oberfläche die innere Oberfläche einer mit ePTFE behandelten Gefäßprothese ist.

13. Verfahren nach Anspruch 12, wobei die Gefäßprothese einen Innendurchmeser von 1 bis 30 mm hat.

14. Material, das in einen Körper eines Lebewesens implantierbar ist und durch das Verfahren nach einem der Ansprüche 1 bis 13 hergestellt wurde.

15. Gefäßprothese, die durch das Verfahren nach Anspruch 12 oder 13 hergestellt wurde.

16. Vorrichtung, die ein Material nach Anspruch 14 aufweist.

17. Vorrichtung nach Anspruch 16, wobei die Vorrichtung zur Verwendung in Kontakt mit einem Gewebe eines Lebewesens vorgesehen ist.

18. Vorrichtung nach Anspruch 17, wobei das Gewebe Blutgewebe ist.

19. Vorrichtung nach Anspruch 17 oder 18, wobei die Vorrichtung ausgewählt ist aus der Gruppe bestehend aus:
einem Implantat, einer Prothese, einem künstlichen Organ, einem Stent, einer Herzklappe, einem während einer extrakorporalen Blutzirkulation mit Blut in Kontakt tretenden Apparat, einem während einer Dialysebehandlung mit Blut in Kontakt tretenden Apparat, oder einem beliebigen anderen Material mit Oberflächen, die mit Blut in Kontakt kommen.

20. Verwendung eines Materials nach Anspruch 14 zur Herstellung einer Vorrichtung zur Prävention von Thrombose.

21. Material, das durch das Verfahren nach einem der Ansprüche 1 bis 13 hergestellt wurde, und das für den Kontakt mit cincm lebenden Gewebe geeignet ist, **dadurch gekennzeichnet, dass** es eine Oberfläche aufweist, die resistent gegenüber neutrophiler Adhäsion, Plättchenadhäsion und -aktivierung ist, die jedoch die Entwicklung von Fibroblasten und Endothelzellen fördert.

22. Material nach Anspruch 21, das plasmabehandeltes ePTFE mit kovalent gepfropftem Phosphorylcholin (PRC) ist.

23. Vorrichtung, die ein Material nach Anspruch 21 oder 22 aufweist.

24. Vorrichtung nach Anspruch 23, die ausgewählt ist aus der Gruppe bestehend aus:
einem Implantat, einer Prothese, einem künstlichen Organ, einem Stent, einer Herzklappe, einem während einer extrakorporalen Blutzirkulation mit Blut in Kontakt tretenden Apparat, einem während einer Dialysebehandlung mit Blut in Kontakt tretenden Apparat, oder einem beliebigen anderen Material mit Oberflächen, die mit Blut in Kontakt kommen.

25. Verwendung eines Materials nach Anspruch 21 oder 22 zur Herstellung einer Vorrichtung, die eine zur Prävention von Thrombose behandelle Oberfläche aufweist.

## Revendications

1. Procédé pour modifier les propriétés de surface d'un matériau adapté pour entrer en contact avec un tissu vivant, consistant à :
- exposer la surface du matériau aux conditions de traitement par plasma afin de créer des groupes réactifs sur ladite surface ;
- activer une molécule avec du phosphorylchlorure (POCI3) pour produire des espèces moléculaires réactives capables de former des liaisons covalentes avec les groupes réactifs créés sur la surface du matériau ; et
- mettre en contact les espèces moléculaires réactives avec les groupes réactifs créés sur la surface du matériau pour former des liaisons covalentes.

2. Procédé selon la revendication 1, dans lequel la surface résultante du matériau résultant est compatible avec le tissu vivant.

3. Procédé selon la revendication 2, dans lequel ledit tissu est un tissu sanguin.

4. Procédé selon la revendication 1, dans lequel
- ledit plasma est du plasma radiofréquence (RF) ammoniacal et lesdits groupes réactifs sont des groupes aminés ;
- ladite molécule est choisie dans le groupe comprenant la choline l'héparine et d'autres molécules connues de l'homme du métier pour leur hémocompatibilité, et ladite espèce moléculaire réactive est le dérivé oxyphosphorodichloré de ladite molécule ; et
- lesdites liaisons covalentes sont des liaisons covalentes du type phosphoamide.

5. Procédé selon la revendication 4, dans lequel lesdites conditions de traitement par plasma comprennent l'application d'une puissance RF comprise entre 5 watts et 500 watts pendant une période de 10 secondes à 30 minutes à une pression de 6,661 Pa (50 mtorr) à 666,1 Pa (5 torr).

6. Procédé selon la revendication 5, dans lequel lesdites conditions de traitement par plasma comprennent l'application d'une puissance RF de 20 watts pendant une période de 250 secondes à une pression de 39,966 Pa (300 mtorr).

7. Procédé selon la revendication 5, dans lequel lesdites conditions de traitement par plasma comprennent l'application d'une puissance RF de 15 watts pendant une période de 100 secondes à une pression de 33,305 Pa (250 mtorr).

8. Procédé selon la revendication 6 ou 7, dans lequel la mise en contact des espèces moléculaires réactives avec les liaisons réactives est effectuée pendant les 2 heures d'exposition de la surface du matériau au traitement par plasma.

9. Procédé selon l'une quelconque des revendications 1 à 8, dans lequel ladite surface dudit matériau adapté pour entrer en contact avec un tissu vivant est un matériau implantable dans un corps d'animal.

10. Procédé selon la revendication 9, dans lequel ladite surface dudit matériau implantable dans un corps d'animal est choisie dans le groupe comprenant :
le polytétrafluoroéthylène expansé (ePTFE) microporeux, le polyétatrafluoroéthylène (PTFE), le polychlorure de vinyle (PVC), les polyéthylènes, les polyesters (tels que le polyéthylène téréphtalate - PET), les polypropylènes, les polyuréthanes, les polycarbonates, les silicones, les PVDF et les matériaux recouverts de polymère, tels que les métaux et céramiques par exemple.

11. Procédé selon la revendication 10, dans lequel ladite surface dudit matériau implantable dans un corps d'animal est du ePTFE.

12. Procédé selon la revendication 11, dans lequel ladite surface est la surface interne d'une prothèse vasculaire traitée avec du ePTFE.

13. Procédé selon la revendication 12, dans lequel ladite prothèse vasculaire a un diamètre interne de 1 à 30 mm.

14. Matériau implantable dans un corps d'animal produit par le procédé selon l'une quelconque des revendications 1 à 13.

15. Prothèse vasculaire produite par le procédé des revendications 12 ou 13.

16. Dispositif comprenant un matériau tel que défini dans la revendication 14.

17. Dispositif selon la revendication 16, dans lequel ledit dispositif doit être utilisé en contact avec un tissu d'animal.

18. Dispositif selon la revendication 17, dans lequel ledit tissu est un tissu sanguin.

19. Dispositif selon la revendication 17 ou 18, dans lequel ledit dispositif est choisi dans le groupe comprenant :
un implant, une prothèse, un organe arrificiel, un stent, une valvule cardiaque, un appareil en contact avec le sang pendant une circulation sanguine extra-corporelle, un appareil en contact avec le sang pendant un traitement de dialyse ou n'importe quel autre matériau ayant des surfaces venant en contact avec le sang.

20. Utilisation d'un matériau tel que défini dans la revendication 14 dans la fabrication d'un dispositif pour prévenir la thrombose.

21. Matériau produit par le procédé selon l'une quelconque des revendications 1 à 13 adapté pour entrer en contact avec un tissu vivant **caractérisé en ce qu'**il possède une surface qui résiste à l'adhérence neutrophile, à l'adhérence et l'activation plaquettaire, mais qui améliore lc développement de fibroblastes et de cellules endothéliales.

22. Matériau selon la revendication 21 qui est un Eptfe traité au plasma avec de la phosphorylcholine (PRC) greffée de manière covalente.

23. Dispositif comprenant un matériau tel que défini dans la revendication 21 ou 22.

24. Dispositif selon la revendication 23, choisi dans le groupe comprenant :
un implant, une prothèse, un organe artificiel, un stent, une valvule cardiaque, un instrument en contact avec le sang pendant une circulation sanguine extra-corporelle, un instrument en contact avec le sang pendant un traitement de dialyse ou n'importe quel autre matériau ayant des surfaces venant en contact avec le sang.

25. Utilisation d'un matériau selon la revendication 21 ou 22 dans la fabrication d'un dispositif ayant une surface traitée pour prévenir la thrombose.
